# EUROPÄISCHE PATENTSCHRIFT

(11) **EP 0 582 928 B1**
(45) Veröffentlichungstag und Bekanntmachung des Hinweises auf die Patenterteilung: **12.05.1999**
(21) Anmeldenummer: 93112345.9
(22) Anmeldetag: 02.08.1993
(51) Int. Cl.: C08G 63/00, C07C 69/00, B01F 17/00, C08G 63/66, C08G 63/68, C09D 17/00, C07C 305/10, C07C 309/17, C08G 69/44, C08G 65/32, C09B 41/00, C09B 67/00, D06P 1/613, D06P 1/52

(54) **Grenzflächenaktive Verbindungen auf Basis modifizierter Rizinusölfettkörper**
Surface active compounds based on modified castor oil fatty substances
Composés tensioactifs à base de corps gras d'huile de ricin modifiés

(30) Priorität: 11.08.1992 DE 4226504
(43) Veröffentlichungstag der Anmeldung: 16.02.1994
(73) Patentinhaber: Clariant GmbH, 65929 Frankfurt am Main (DE)
(72) Erfinder: Uhrig, Heinz, D-61449 Steinbach/Taunus (DE); Münkel, Albert, D-65835 Liederbach (DE)

(56) Entgegenhaltungen:
- EP-A- 0 001 206
- EP-A- 0 032 188
- DE-A- 2 726 439
- FR-A- 2 313 116
- FR-A- 2 437 421
- US-A- 3 332 793
- DATABASE WPI Week 8444, Derwent Publications Ltd., London, GB; AN 84-273038 & JP-A-59 168 081 (NEW JAPAN CHEM. ET AL.) 21. September 1984
- CHEMICAL ABSTRACTS, vol. 111, no. 22, 27. November 1989, Columbus, Ohio, US; abstract no. 196563, & JP-A-1 111 012 (ASAHI CHEM. IND. CO. LTD.) 27. April 1989
- DATABASE WPI Week 8114, Derwent Publications Ltd., London, GB; AN 81-24419D & JP-A-56 014 591 (MIYOSHI YUSHI KK) 12. Februar 1981
- RESEARCH DISCLOSURE, Bd.191, März 1980, HAVANT GB Seite 110 '19113 New finishes'

## Beschreibung

Die Erfindung liegt auf dem Gebiet grenzflächenaktiver Mittel. Bei der Herstellung von Dispersionen und Präparationen von Farbmitteln, beispielsweise Dispersionsfarbstoffen, vorzugsweise jedoch anorganischen und organischen Pigmenten, für Anwendungen in wäßrigen und organischen Medien wird eine Vielzahl nichtionischer, anionischer und auch kationischer Tenside benutzt. Die Art der Tenside hat einen wesentlichen Einfluß auf die Feinverteilung und damit auf die Farbstärke der Farbmittel in den jeweiligen Anwendungsmedien. Außerdem werden Viskosität, Glanz und Lagerstabilität maßgeblich von der Art der Tenside beeinflußt. Auch bei der Herstellung von Azofarbstoffen aus schwer löslichen Kupplungskomponenten werden üblicherweise Tenside eingesetzt, die eine gute Verteilung der Kupplungskomponente in dem Reaktionsmedium und eine vollständige Reaktion bei der Azokupplung ermöglichen. Ebenso werden durch die oberflächenaktiven Eigenschaften solcher Tenside die coloristischen Eigenschaften in den verschiedenen Anwendungsmedien auf dem Gebiet der Druckfarben wesentlich beeinflußt. Weiterhin werden beispielsweise zum Färben von Leder neben bekannten Neutralsalzen synthetischer Hilfsstoffe anionische, kationische und amphotere Hilfsmittel, wie unter anderem verschiedene Amine, Aminharze, modifizierte Eiweißhydrolysate und Kondensationsprodukte von Polysäuren mit Aminen, verwendet (Ullmann, Encyklopädie der technischen Chemie, 4. Aufl., 1978, Band 16, Seite 150).

Aus der DE-A-27 05 441 (AU 77/21701) sind nichtionische grenzflächenaktive Mittel als Solubilisierungsmittel bekannt, die man durch Oxethylierung von natürlichem Rizinusöl oder hydriertem Rizinusöl erhält.

Nach der DE-A-2 504 136 (GB-A-15 32 506) werden für die Zubereitung von Emulsionskonzentraten von wasserunlöslichen Bioziden phenyl-, tolyl- und benzylmodifizierte Rizinusöl-Oxethylate verwendet.

Nach der EP-A1-0 032 188 (US-PS 4 597 906 und US-PS 50 08 442) werden wasserlösliche oberflächenaktive Verbindungen auf Basis von arylierten Fettkorpern als Kupplungshilfsmittel für Azopigmente und als Dispergiermittel für Dispersionsfarbstoffe hergestellt.

In der EP-A1-61 683 (US-PS 4 371 683) wird für die Herstellung von härtbaren Klebemitteln neben aliphatischen Polyalkylenoxidderivaten und Polystyrolen auch Rizinusöl genannt.

In den amerikanischen Patentschriften US-PS 1 779 367, 1 785 930, 1 978 533, 2 027 467, 2 107 610 und 2 153 511 werden Alkydharze auf Basis von natürlichen und modifizierten Rizinusölen mit Bernsteinsäure, Phthalsäure und Glyzerin beschrieben.

Weiterhin werden in der US-PS 3 560 419 unter Mitverwendung von Rizinusöl-Oxethylaten Phosphorsäureester als Viskosezusatzöle und Präparationsmittel für die Herstellung von Polyamidspinnfasern genannt.

Textilhilfsmittel, wie beispielsweise Netz-, Färberei- und Mercerisierhilfsmittel auf Basis von niedrig- und hochsulfatierten oder sulfonierten Rizinusölen sowie Rizinolsäuremonoestern sind als Türkischrotöle, Monopolseifen und Prestabitöle in Lindner, "Tenside, Textilhilfsmittel und Waschrohstoffe", 2. Auflage, 1964, Band 1, Seite 624 bis 633 beschrieben.

Keines der in den vorstehend genannten Druckschriften beschriebenen Produkte ist jedoch geeignet, die Fließfähigkeit von Druckfarben sowie die Flockungsstabilität von Außenanstrichfarben entscheidend zu verbessern, ohne andere Parameter, wie Farbstärke, Glanz, Farbton und Dispergierbarkeit, nachteilig zu beeinflussen.

Der vorliegenden Erfindung lag die Aufgabe zugrunde, grenzflächenaktive Mittel zur Verfügung zu stellen, die zur Herstellung gut fließfähiger und flockungsbeständiger Feststoffdispersionen, vorzugsweise Farbmitteldispersionen, für den Außenanstrich geeignet und weitgehend frei von den vorstehend genannten Nachteilen sind.

Gegenstand der vorliegenden Erfindung sind neue Verbindungen auf der Basis eines Rizinusöles oder einer Rizinolsäure, bestehend aus
a) 1 bis 10, vorzugsweise 1 bis 5, Einheiten der Formel (la) 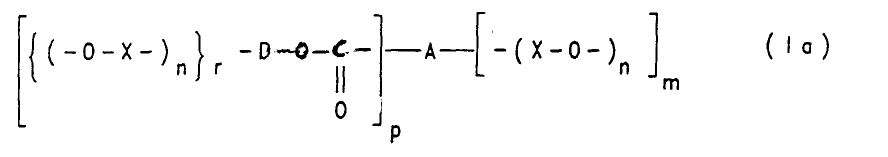
b) 1 bis 72, vorzugsweise 1 bis 23, insbesondere 2 bis 9, monovalenten Resten der Formel (Ib)

   -Z (Ib),

   und im Falle des Vorhandenseins von zwei oder mehreren Einheiten der Formel (la) zusätzlich aus
c) 1 bis 9, vorzugsweise 1 bis 4, divalenten Gruppen der Formel (Ic)

   -CO-E-CO- (Ic),
wobei jede der gezeigten freien Valenzen in den Formeleinheiten (la) so definiert ist, daß sie unabhängig voneinander mit jeweils einer Formeleinheit (Ib) oder einer Valenz der Formeleinheit (Ic) direkt verbunden ist, und wobei in den Formeleinheiten (la) bis (Ic)
- A: für den Rest eines natürlichen oder modifizierten Rizinusöles oder für einen 12-Hydroxy-9-octadecenyl-(cis)- oder 12-Hydroxy-octadecyl-Rest steht;
- D: für eine direkte Bindung oder für einen zweiwertigen Rest eines geradkettigen oder verzweigten 2- bis 6-wertigen Alkohols mit 2 bis 8 C-Atomen oder für einen zweiwertigen Rest eines Alkylolamins HO-(CH₂)ᵥ-[NH-(CH₂)ᵤ]ₐ-NH₂, 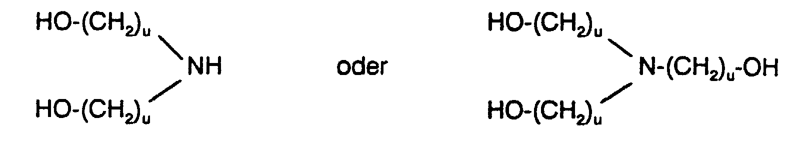 steht,
worin a für eine ganze Zahl von 0 bis 4,
u und v gleich oder verschieden sind und für eine ganze Zahl von 2 bis 14, vorzugsweise 2 bis 3, stehen
und wobei jede oder ein Teil der OH-, NH- oder NH₂-Gruppen oxalkyliert ist;
- E: für einen geradkettigen, verzweigten oder cyclischen aliphatischen Rest mit 0 bis 20, vorzugsweise 1 bis 10, Kohlenstoffatomen; für einen aromatischen Rest mit 6 bis 10 Kohlenstoffatomen; für einen Alkenylrest auf Basis einer dimerisierten ungesättigten C₂₈-C₇₂-Fettsäure;
oder für eine Gruppe der Formeln -CH=CH-, -CH₂CH(SO₃M)- oder -CH(SO₃M)CH₂- steht, worin M ein Kation oder ein Rest der Formel -(X-O-)ₙH ist;
- X: für eine Gruppe der Formeln -CH₂CH₂-, -CH(CH₃)CH₂ und -CH₂CH(CH₃)- oder für eine Kombination davon steht;
- Z: für gleiche oder verschiedene Reste Z¹ bis Z⁶ steht, worin
- Z¹: Wasserstoff,
- Z²: einen Acylrest der Formel R¹-CO-, in der R¹ einen Phenyl- oder Naphthyl rest bedeutet,
- Z³: einen Acylrest einer unmodifizierten oder modifizierten natürlichen Harzsäure,
- Z⁴: einen Acylrest einer Di- oder Tricarbonsäure auf Basis einer di- oder trimerisierten C₂₈-C₇₂-Fettsäure oder einen Acylrest einer gesättigten oder ungesättigten C₈-C₂₂-Fettsäure,
- Z⁵: gleiche oder verschiedene Reste der Formeln -CO-(CH₂)_{q}-COOM, -CO-CH=CH-COOM, -CO-CH₂-CH(SO₃M)-COOM, -CO-CH(SO₃M)-CH₂-COOM und -OC-C₆H₄-COOM bedeuten, worin q für eine ganze Zahl von 0 bis 10 steht,
- Z⁶: -SO₃M,
worin M für Wasserstoff; ein Alkalimetall; ein Äquivalent eines Erdalkalimetalls; einen Oxalkylrest der Formel (X-O-)ₙH; eine Ammoniumgruppe, die unsubstituiert oder durch ein bis vier C₁-C₅-Alkylreste oder ein bis vier C₂-C₅-Alkylolreste substituiert ist; eine aus Ammoniak oder aus C₁-C₅-Alkylaminen oder C₂-C₅-Alkylolaminen durch Anlagerung von 1 bis 150, vorzugsweise 5 bis 30, Ethylenoxid- oder Propylenoxideinheiten oder einer Kombination von Ethylenoxid- und Propylenoxideinheiten erhaltene Ammoniumgruppe; oder eine Gruppe der Formel (II) 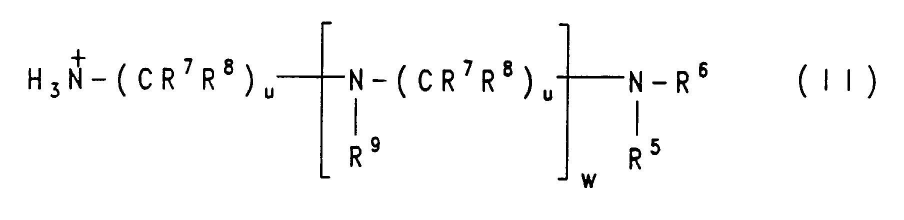 ist, worin R⁹, R⁵ und R⁶ gleich oder verschieden sind und für ein Wasserstoffatom oder eine Hydroxyalkylgruppe mit 1 bis 6 C-Atomen, vorzugsweise 2 bis 3 C-Atomen, und R⁷ und R⁸ gleich oder verschieden sind und für Wasserstoff oder Methyl stehen, u jeweils gleich oder verschieden ist und eine ganze Zahl von 2 bis 14, bevorzugt 2 bis 3, bedeutet und w für eine ganze Zahl von Null bis 25, bevorzugt von Null bis 5, steht; oder worin M für eine Gruppe der Formel (III) steht 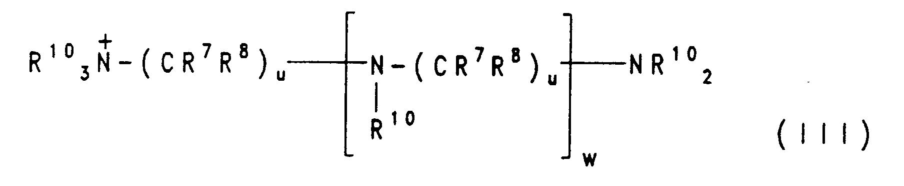 worin R¹⁰ die Gruppe H-(O-X-)_{y} bedeutet, worin X die vorstehend genannten Bedeutungen hat und y eine ganze Zahl von 1 bis 100, vorzugsweise 1 bis 30, ist,
- m: im Falle, daß A ein Rizinolsäurerest ist, für die Zahl 1, und im Falle, daß A ein Rizinusölrest ist, für eine Zahl von 1 bis 2,
- n: für eine Zahl von 1 bis 250, vorzugsweise 5 bis 125, besonders bevorzugt 10 bis 80,
- p: im Falle, daß A ein Rizinusöl ist, für die Zahl 0, und im Falle, daß A ein Rizinolsäurerest ist, für die Zahl 1 und
- r: für eine Zahl von 1 bis 6, vorzugsweise 1 bis 4, stehen,
und wobei mindestens ein Rest Z aus der Gruppe, Z³ ist; oder wobei mindestens zwei Einheiten der Formel (la) über eine divalente Gruppe der Formel (Ic) miteinander verknüpft sind und Z die Bedeutung von Z¹ bis Z⁶ hat.

Unter modifiziertem Rizinusöl wird ein einfach oder mehrfach hydriertes Rizinusöl verstanden. Unter modifizierter Rizinolsäure wird eine hydrierte Rizinolsäure verstanden.

Unter "Rizinusölfettkörper" wird natürliches oder modifiziertes Rizinusöl oder natürliche oder modifizierte Rizinolsäure verstanden.

Von besonderem Interesse sind erfindungsgemäß solche Verbindungen, die 1 bis 5 Einheiten der Formel (la) enthalten, worin
- A: für den Rest eines natürlichen oder modifizierten Rizinusöles steht und p daher die Zahl 0 bedeutet,
- X: für -CH₂CH₂- und
- n: für eine ganze Zahl von 5 bis 125, vorzugsweise 10 bis 80, stehen.

Von besonderem Interesse sind ferner solche Verbindungen, die 1 bis 5 Einheiten der Formel (Ia) enthalten, worin
- A: für einen 12-Hydroxy-9-octadecenyl-(cis)- oder 12-Hydroxy-octadecyl-Rest steht, p die Zahl 1 bedeutet,
- D: für einen zweiwertigen Rest eines geradkettigen oder verzweigten 2- bis 6-wertigen Alkohols mit 2 bis 6 C-Atomen oder eines C₂-C₃-Alkylolamins, C₂-C₃-Alkylol-C₂-C₃-alkylendiamins, C₂-C₃-Alkylol-di-C₂-C₃-alkylen-triamins oder C₂-C₃-Alkylol-tri-C₂-C₃-alkylen-tetramins,
- X: für -CH₂CH₂- und
- n: für eine ganze Zahl von 5 bis 125, vorzugsweise 10 bis 80, stehen.

Von besonderem Interesse sind weiterhin solche erfindungsgemäßen Verbindungen, die 2 bis 9 monovalente Reste der Formel (Ib) enthalten, worin Z für gleiche oder verschiedene Reste der Formeln Z², Z³, Z⁴ und Z⁵, vorzugsweise Z³ und Z⁵, steht.

Von besonderem Interesse sind ferner solche erfindungsgemäßen Verbindungen, die 1 bis 4 divalente Gruppen der Formel (Ic) enthalten, worin E für einen geradkettigen, verzweigten oder cyclischen aliphatischen Alkylenrest mit 2 bis 10 Kohlenstoffatomen, vorzugsweise Ethylen, Butylen, Pentylen, Hexylen und Decylen; für einen Phenylenrest; oder für eine Gruppe der Formeln -CH=CH-, -CH₂CH(SO₃M)- oder -CH(SO₃M)CH₂-, worin M ein Kation ist, steht.

Gegenstand der vorliegenden Erfindung ist auch ein Verfahren zur Herstellung der erfindungsgemäßen Verbindungen, dadurch gekennzeichnet, daß man
a) natürliches oder modifiziertes Rizinusöl mit mindestens einer den Resten Z², Z³, Z⁴, Z⁵, Z⁶ und -CO-E-CO- zugrundeliegenden Säure oder einem reaktiven Derivat dieser Säure teilweise oder vollständig verestert, wobei der erhaltene Ester noch mindestens eine freie Hydroxygruppe enthät; oder daß man natürliche oder hydrierte Ricinolsäure mit einem dem Rest D zugrundeliegenden Alkohol oder Alkylolamin verestert;
b) anschließend die freien Hydroxygruppen oder Carboxygruppen der nach a) erhältlichen Verbindungen mit Ethylenoxid oder Propylenoxid oder beiden Epoxiden hintereinander oder einem Gemisch beider Epoxide oxalkyliert;
c) anschließend gegebenenfalls die nach b) erhaltenen Oxalkylate mit
   c1) mindestens einer den Acylresten Z², Z³, Z⁴, Z⁵ und -CO-E-CO- zugrundeliegenden Carbonsäure oder einem reaktiven Derivat dieser Carbonsäure, vorzugsweise einem Anhydrid, vollständig verestert oder teilverestert oder mit mehreren der vorstehend genannten Carbonsäuren oder deren reaktiven Derivaten mischverestert,
   c2) und vorhandene Maleinsäurehalbestergruppen gegebenenfalls mit einem Sulfit oder mit schwefliger Säure umsetzt
   c3) oder die nach b) erhaltenen Oxalkylate mit einem dem Rest Z⁶ zugrundeliegenden Sulfatierungsmittel, vorzugsweise Schwefelsäure, Chlorsulfonsäure, Amidosulfonsäure oder Schwefeltrioxid, sulfatiert; und
d) im Falle, daß in c) ein Rest der Formel Z⁵ oder Z⁶ eingeführt wurde, gegebenenfalls mit einer dem Rest M zugrundeliegenden Base in das entsprechende Salz oder Oxalkylat überführt.

Im Falle, daß als Ausgangsprodukt eine natürliche oder modifizierte Rizinolsäure eingesetzt wird, ist es zweckmäßig, die freie Carboxylgruppe der Rizinolsäure durch einen dem Rest D zugrundeliegenden 2- bis 6-wertigen Alkohol oder ein Alkylolamin zu verestern. Es ist vorteilhaft, die besagte Veresterung vor dem Oxalkylierungsschritt b) durchzuführen.
Zur Veresterung der freien Carboxygruppen der Rizinolsäure sind von besonderem Interesse: Glyzerin, Diglyzerin, Polyglyzerine, 1,2,4-Butantriol, Butandiol-(1,4), Glykol, Polyglykole, 1,1,1-Trimethylolethan, 1,1,1-Trimethylolpropan, Pentaaerythrit, 2,4-Dihydroxy-3-methylol-pentan, Hexantriol, Sorbit, Anhydrosorbit, Hexit oder Mannit sowie Mono-, Di- und Triethanolamin und weiterhin Alkylolamine, wie N-(2-Hydroxyethyl)-1,2-diaminoethan, N-(2-Hydroxyethyl)-diethylentriamin, N-(2-Hydroxyethyl)-triethylentetramin und 2-Aminoethanol.

Die Veresterung mit den besagten mehrwertigen Alkoholen oder Alkylolaminen erfolgt im Molverhältnis Alkohol oder Alkylolamin : Rizinolsäure von 1:1 bis 4:1, vorzugsweise 1:1 bis 2:1, bei einer Temperatur von 180 bis 300°C, vorzugsweise bei 200 bis 270°C, gegebenenfalls unter Zusatz eines Schleppmittels, beispielsweise eines aromatischen Kohlenwasserstoffes oder Chlorkohlenwasserstoffes. Als Katalysatoren können beispielsweise Benzolsulfonsäure, p-Toluolsulfonsäure, Borsäure, Zinnpulver oder Schwefelsäure verwendet werden. Zinnpulver wird in einer Menge von 1 bis 4 Gew.-%, die übrigen genannten Säuren werden in einer Menge von 0,1 bis 2 Gew.-%, vorzugsweise 0,5 bis 1 Gew.-%, jeweils bezogen auf die umzusetzende Verbindung, eingesetzt.

Der der Gruppe A zugrundeliegende Rizinusölfettkörper ist beispielsweise handelsübliches Rizinusöl, bestehend im wesentlichen aus einem Glycerid der Rizinolsäure, Ölsäure, Linolsäure und Stearinsäure, oder ist einfach oder mehrfach hydriertes Rizinusöl, hydrierte Rizinolsäure. Natürliches Rizinusöl enthält freie Hydroxylgruppen und olefinische Doppelbindungen.

Die dem Acylrest Z² zugrundeliegenden aromatischen Carbonsäuren sind beispielsweise Benzoesäure, 1-Naphthoesäure oder 2-Naphthoesäure.

Die dem Acylrest Z³ zugrundeliegenden Harzsäuren sind natürliche oder modifizierte Harzsäuren, beispielsweise Abietinsäure, Dehydroabietinsäure, Dihydroabietinsäure, Tetrahydroabietinsäure, Laevopimarsäure, Dextropimarsäure und Isodextropimarsäure, wie sie in handelsüblichen Kolophoniumarten vorliegen, weiterhin disproportionierte, hydrierte und dimerisierte Harzsäuren.

Die dem Acylrest Z⁴ zugrundeliegenden Fettsäuren sind dimerisierte oder trimerisierte Fettsäuren mit 28 bis 72, insbesondere 36 bis 54, Kohlenstoffatomen, sowie gesättigte oder ungesättigte C₈-C₂₂-Fettsäuren, beispielsweise Octansäure, Pelargonsäure, Caprinsäure, Undecansäure, Laurinsäure, Tridecansäure, Myristinsäure, Pentadecansäure, Palmitinsäure, Heptadecansäure, Stearinsäure, Nonadecansäure, Arachinsäure, Behensäure, 10-Undecensäure, Lauroleinsäure, Myristoleinsäure, Palmitoleinsäure, 6c- und 6t-Octadecensäure, Elaidinsäure, Ölsäure, Linolsäure und Linolensäure, insbesondere geradzahlige Fettsäuren oder Hydroxyfettsäuren mit jeweils 8 bis 20 C-Atomen, beispielsweise die entsprechenden vorstehend genannten geradzahligen Fettsäuren sowie insbesondere ihre aus Naturprodukten gewonnenen Gemische, wie Tallölfettsäure, Talgfettsäure, Kokosölfettsäure, Palmölfettsäure und Leinölfettsäure, vorzugsweise die genannten Fettsäuren mit 12 bis 18 C-Atomen.

Die dem Acylrest Z⁵ zugrundeliegenden Säuren oder Carbonsäureanhydride sind beispielsweise Maleinsäure, Maleinsäureanhydrid, Fumarsäure, Phthalsäure, Phthalsäureanhydrid, Isophthalsäure, Terephthalsäure, Oxalsäure, Malonsäure, Bernsteinsäure, Bernsteinsäureanhydrid, Glutarsäure, Glutarsäureanhydrid, Adipinsäure, Pimelinsäure, Korksäure, Acelainsäure, Sebacinsäure und Sulfobernsteinsäure, vorzugsweise Bernsteinsäure, Phthalsäure, Terephthalsäure, Maleinsäure und Fumarsäure, insbesondere Sulfobernsteinsäure.

a) Die Veresterung der Hydroxylgruppen der vorstehend genannten Rizinusöle und Rizinolsäuren mit den den Acylresten Z², Z³, Z⁴ und Z⁵ zugrundeliegenden Carbonsäuren erfolgt im molaren Verhältnis Rizinusölfettkörper: Carbonsäure(anhydrid) von 1:1 bis 1:9, vorzugsweise 1:1 bis 1:4, und wird nach an sich üblichen Veresterungsmethoden durchgeführt. Die einzuhaltende Reaktionstemperatur liegt dabei in der Regel zwischen 20°C und 240°C je nach Veresterungsmethode. Bevorzugt wird zur Erhöhung der Ausbeute die Veresterung in einem inerten organischen Lösungsmittel durchgeführt, das als Schleppmittel zum Entfernen des Reaktionswassers geeignet ist. Beispielsweise kann die Veresterung in Xylol als organischem Lösungsmittel und in Gegenwart von sauren Katalysatoren bei einer Temperatur von 130 bis 220°C durchgeführt werden. Als saure Katalysatoren sind beispielsweise Säuren und Lewissäuren wie Benzolsulfonsäure, p-Toluolsulfonsäure, Borsäure, Zinnpulver, Zinkchlorid oder Schwefelsäure in den vorstehend angegebenen Konzentrationen geeignet.

Werden die vorstehend genannten Rizinusöle und Rizinolsäuren mit einer dem Acylrest -OC-E-CO- zugrundeliegenden Dicarbonsäure verestert, so können 2 bis 10, vorzugsweise 2 bis 5, Rizinusöl- oder Rizinolsäure-Einheiten, die gegebenenfalls mit einer oder mehreren Resten Z², Z³, Z⁴ oder Z⁶ teilverestert sind, miteinander durch Gruppen der Formel -OC-E-CO- verknüpft werden. Die zur Verknüpfung führende Veresterung wird mit einer oder mehreren Dicarbonsäuren der Formel HOOC-E-COOH im Molverhältnis Rizinusölfettkörper : Dicarbonsäure von 2:1 bis 10:9, vorzugsweise 2:1 bis 5:4, durchgeführt. Als Dicarbonsäuren eignen sich zur Verknüpfung vorzugsweise aliphatische Dicarbonsäuren mit 3 bis 12 Kohlenstoffatomen, insbesondere Malonsäure, Bernsteinsäure, Glutarsäure, Adipinsäure, 1,5-Pentandicarbonsäure, 1,6-Hexandicarbonsäure oder 1,10-Decandicarbonsäure, aber auch beispielsweise Cyclohexan-1,4-dicarbonsäure und aromatische Dicarbonsäuren, wie Phthalsäure oder Terephthalsäure, sowie dimerisierte ungesättigte C₂₈-C₇₂-Fettsäuren. Anstelle der Dicarbonsäuren können zur Veresterung auch Anhydride, beispielsweise Maleinsäureanhydrid, Bernsteinsäureanhydrid oder Phthalsäureanhydrid sowie reaktive Dicarbonsäurederivate eingesetzt werden, beispielsweise im Falle von Umesterungsreaktionen Alkylester der genannten Dicarbonsäuren.

Es ist auch möglich, teilveresterte oder verknüpfte Rizinusölfettkörper mit den den Acylresten Z², Z³, Z⁴ und Z⁵ zugrundeliegenden Carbonsäuren oder Anhydriden weiter zu verestern. Diese Veresterung erfolgt nach bekannten Verfahren bei Temperaturen von 130 bis 220°C, vorzugsweise bei 150 bis 180°C, in Gegenwart von anorganischen oder organischen Säuren oder von Lewissäuren, wie Zinkchlorid, Benzolsulfonsäure, p-Toluolsulfonsäure, Borsäure, Zinnpulver oder Schwefelsäure in den vorstehend angegebenen Konzentrationen. Das sich dabei bildende Reaktionswasser wird gegebenenfalls unter Zusatz eines Schleppmittels, beispielsweise eines Kohlenwasserstoffes oder Chlorkohlenwasserstoffes, durch Abdestillieren entfernt. Bei Verwendung von Dicarbonsäureanhydriden erfolgt eine Halbveresterung schon bei 0 bis 150°C, vorzugsweise bei 40 bis 120°C, gegebenenfalls in Anwesenheit von Alkalihydroxiden, wobei die Konzentration der Alkalihydroxide 0,1 bis 1,0 Gew.-%, bezogen auf die Gesamtmischung, beträgt. Im Falle von Maleinsäureanhydrid, Bernsteinsäureanhydrid und Phthalsäureanhydrid ist es wegen der Sublimationsneigung vorteilhaft, in Druckgefäßen unter einem Überdruck von 0,2 bis 1,0 bar Stickstoff oder Luft zu arbeiten und für kräftiges Durchmischen zu sorgen, da zu Beginn der Reaktion die geschmolzenen Anhydride mit den verschiedenen Rizinusverbindungen nur in geringem Maße mischbar sind.

b) Die Oxalkylierung der genannten Rizinusöle oder Rizinolsäuren, deren Veresterungs- und Arylierungsprodukte sowie der durch Verknüpfung zweier oder mehreren Struktureinheiten der Formel (la) erhältlichen Verbindungen erfolgt nach üblichen Methoden, vorzugsweise mit Alkalihydroxiden oder -alkoxiden als Katalysatoren bei 100 bis 200°C, bevorzugt bei 140 bis 180°C. Die Menge Ethylenoxid und/oder Propylenoxid wird so bemessen, daß eine stabile Emulgierbarkeit oder eine völlige Löslichkeit der Oxalkylate in Wasser erreicht wird. Bevorzugt werden an jede freie Carboxy- und Hydroxygruppe je 1 bis 250, vorzugsweise 5 bis 125, insbesondere 10 bis 80, Mol Ethylenoxid und/oder Propylenoxid angelagert. Die Menge des angelagerten Alkylenoxids bemißt sich auch nach dem beabsichtigten Einsatzzweck und damit dem angestrebten Grad der Hydrophilie.

Als Alkalihydroxid bevorzugt sind Kaliumhydroxid oder Natriumhydroxid, als Alkalialkoxid Natriummethanolat oder -ethanolat; die Konzentration des Katalysators ist zu Beginn der Oxalkylierung vorzugsweise 0,05 bis 2,0 Gew.-%, bezogen auf die zu oxalkylierende Verbindung. Die Oxalkylierung kann unter Atmosphärendruck oder in Druckgefäßen bei 1 bis 10, vorzugsweise 2 bis 4, bar mit Propylenoxid oder bevorzugt Ethylenoxid oder Mischungen von beiden durchgeführt werden, wobei das Alkylenoxid gasförmig oder flüssig zugeführt wird. Gegebenenfalls wird die Reaktionsmischung nach Beendigung der Oxalkylierung neutralisiert, beispielsweise mit Essigsäure.

Die Verknüpfung zweier oder mehrerer der vorstehend genannten oxalkylierten Verbindungen mit Gruppen der Formel -CO-E-CO- (Ic) kann in einer oder zwei Reaktionsstufen durch Veresterung mit den vorstehend genannten Dicarbonsäuren oder deren Anhydriden durchgeführt werden, wobei vorzugsweise ein Verhältnis von Oxalkylaten zu Dicarbonsäure von 2:1 bis 10:9 gewählt wird. Im Fall, daß ein teilverestertes Oxalkylat nur noch eine freie Hydroxygruppe besitzt, ist ein Molverhältnis von 2:1 sinnvoll.

c1) Gegebenenfalls werden in einer, zwei oder drei weiteren Reaktionsstufe(n) die freien endständigen Hydroxygruppen der oxalkylierten Rizinusöl- oder Rizinolsäureverbindungen teilweise oder vollständig mit mindestens einer den Acylresten Z², Z³, Z⁴, Z⁶ und -OC-E-CO- zugrundeliegenden Carbonsäure oder ihrem Anhydrid umgesetzt, wobei die molaren Mengenverhältnisse von Carbonsäure(anhydrid) : Oxalkylat 1:1 bis 9:1, vorzugsweise 1:1 bis 4:1, sind.

Die Veresterung der oxalkylierten Rizinusöl- oder Rizinolsäure-Verbindungen mit den aromatischen Carbonsäuren, natürlichen oder modifizierten Harzsäuren oder einfachen, di- oder trimerisierten Fettsäuren wird nach an sich üblichen Veresterungsmethoden durchgeführt. Die Reaktionstemperatur liegt in der Regel zwischen 20°C und 240°C, je nach Veresterungsmethode. Bevorzugt wird zur Erhöhung der Ausbeute die Veresterung in einem inerten organischen Lösemittel durchgeführt, das als Schleppmittel zum Entfernen des Reaktionswasser geeignet ist. Vorzugsweise wird die Veresterung in Xylol als organischem Lösemittel und in Gegenwart eines sauren Katalysators bei einer Temperatur von 130 bis 220°C durchgeführt. Als saure Katalysatoren können Säuren und Lewissäuren, wie Benzolsulfonsäure, p-Toluolsulfonsäure, Borsäure, Zinnpulver, Zinkchlorid und Schwefelsäure in den vorstehend angegebenen Konzentrationsbereichen verwendet werden.

Die Veresterung der oxalkylierten Rizinusöl- oder Rizinolsäure-Verbindungen oder deren Teilveresterungsprodukte mit den genannten aromatischen Carbonsäuren oder Harzsäuren kann alternativ auch durch Umesterung unter Einsatz der entsprechenden Alkylester, vorzugsweise Methylester, der genannten Säuren in Gegenwart von 0,1 bis 1,0 Mol-Äquivalenten Alkalialkanolat, vorzugsweise Natriummethanolat, bei 150 bis 200°C, vorzugsweise 160 bis 190°C, unter Abdestillieren des freiwerdenden Alkanols, vorzugsweise Methanols, erfolgen.

Zur Herstellung von Mischestern kann ein Gemisch von Harzsäuren, Fettsäuren und/oder aromatischen Carbonsäuren oder deren reaktiven Derivaten eingesetzt werden, oder die Veresterung erfolgt durch mindestens zwei hintereinander ablaufende Teilveresterungsschritte.

Die erfindungsgemäßen nicht-ionogenen aromatischen Carbonsäureester, Harzsäureester oder Fettsäureester der oxalkylierten Rizinusölfettkörper sind als solche wertvolle oberflächenaktive Mittel und können im Sinne der Erfindung eingesetzt werden.

Zur Einführung von anionischen Estergruppen werden erfindungsgemäß solche Dicarbonsäuren und Anhydride eingesetzt, die dem Rest Z⁵ zugrundeliegen.

Im Falle der Anhydride geschieht die Veresterung durch Mischen und Verrühren mit den oxalkylierten Rizinusölfettkörpern bei 10 bis 120°C, bevorzugt bei 40 bis 80°C, in Anwesenheit von Alkalihydroxiden. Die Konzentration der Alkalihydroxide beträgt zweckmäßigerweise 0,1 bis 1,0 Gew.-%, bezogen auf die Gesamtmischung. Im Falle von Maleinsäureanhydrid ist es wegen der Sublimationsneigung vorteilhaft, in Druckgefäßen bei einem Überdruck von 0,2 bis 1,0 bar Stickstoff oder Luft zu arbeiten und für kräftiges Durchmischen zu sorgen, da zu Beginn der Reaktion das geschmolzene Maleinsäureanhydrid mit den teilveresterten Oxalkylaten schlecht mischbar ist.

c2) Im Fall von eingeführten Maleinsäurehalbestergruppen ist es außerdem vorteilhaft, diese Halbestergruppen in die entsprechenden Sulfobernsteinsäurehalbestergruppen zu überführen. Dies gelingt beispielsweise durch Zugabe wäßriger Lösungen von Sulfiten oder Hydrogensulfiten zu den Verbindungen, die Maleinsäurehalbestergruppen aufweisen. Pro Mol Maleinsäurehalbestergruppe werden 1,0 bis 1,5, vorzugsweise 1,0 bis 1,1 Mol, schweflige Säure in Form von Alkali- oder Erdalkalisulfiten oder -hydrogensulfiten oder -pyrosulfiten eingesetzt. Die Umsetzung wird in der Regel in Gegenwart von etwa 50 bis 85 Gew.-% Wasser, bezogen auf die gesamte Lösung oder Mischung, durchgeführt. Die Wassermenge ist abhängig von der Löslichkeit der zugrundeliegenden Sulfobernsteinsäurehalbestersalze und der Viskosität der Lösungen. Die Reaktionstemperatur bei der Umsetzung von Sulfiten mit den Maleinsäurehalbesterverbindungen beträgt in der Regel 20 bis 100°C, insbesondere 40 bis 80°C.

c3) Zur Einführung von SO₃H-Gruppen werden die nach b) erhältlichen Oxalkylate mit 1 bis 9 Mol, vorzugsweise 1 bis 4 Mol, eines Sulfatierungsmittels, beispielsweise Schwefelsäure, Amidosulfonsäure oder Chlorsulfonsäure oder entsprechenden Anhydriden, pro Mol Oxalkylat umgesetzt. Während bei der Sulfatierung mit Amidosulfonsäure die Ammoniumsalze der Schwefelsäurehalbester gebildet werden, entstehen bei der Ausführungsform mit gasförmigem Schwefeltrioxid in Mischungen mit Inertgas sowie auch bei der Sulfatierung mit Chlorsulfonsäure Schwefelsäurehalbester in der Säureform, aus denen nach d) durch Neutralisation mit entsprechenden anorganischen oder organischen Basen Salze hergestellt werden können. Zu dieser Neutralisation werden bevorzugt die Alkalihydroxide eingesetzt, die zu den sehr gut wasserlöslichen Alkalisalzen der erfindungsgemäßen Schwefelsäurehalbester führen.

Gegenstand der Erfindung sind auch solche Ausführungsformen, bei denen im Anschluß an den Reaktionsschritt c) ein oder mehrere Oxalkylierungsschritte analog dem Reaktionsschritt b) und gegebenenfalls ein oder mehrere Veresterungsschritte analog dem Reaktionsschritt c) durchgeführt werden.

d) Die nach c) veresterten oxalkylierten Rizinusölfettkörper werden vorteilhaft mit 1 bis 9, vorzugsweise 1 bis 4, Mol einer M zugrundeliegenden Base pro Mol Rizinusölfettkörper aus c) neutralisiert, insbesondere mit Aminen der Formeln (II) oder (III). Die Umsetzung zu Aminsalzen erfolgt bei einer Temperatur von 20 bis 130°C, vorzugsweise bei 40 bis 90°C, insbesondere bei 70 bis 80°C. Als Aminoverbindungen kommen beispielsweise in Betracht 1,2-Diaminoethan, 1,3-Diaminopropan, 1,2-Diaminopropan, 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,12-Diaminododecan, Diethylentriamin, Dipropylentriamin, Triethylentetramin, Dipropylentetramin, Tetraethylenpentamin, Tetrapropylenpentamin, Pentaethylenhexamin, Pentapropylenhexamin, Hexaethylenheptamin, Hexapropylenheptamin, Heptaethylenoctamin, Heptapropylenoctamin, 1,3-Diamino-2,2-dimethyl-propan, 1,2-Diamino-2-methyl-propan, 1,3-Diamino-2-methyl-propan, 2,5-Diamino-2,5-dimethylhexan, N-(2-amino-ethyl)-1,3-propylendiamin und N,N'-bis-(3-aminopropyl)ethylendiamin.

Während sich die Sulfite besonders zur Bildung der Dialkalisalze der Sulfobernsteinsäurehalbester eignen, ist es bei der Anlagerung von Hydrogensulfiten möglich, durch Neutralisation mit Basen den Grad der Hydrophilie zusätzlich zu beeinflussen. Als Basen kommen in Betracht Ammoniak, C₁-C₅-Alkylamin, C₂-C₅-Alkylolamine, Amine der Formel (II) oder (III) oder deren Alkylenoxidaddukte, wobei pro Mol Amin oder Alkylolamin bis zu etwa 150 Mol Ethylenoxid oder Propylenoxid oder beide Alkylenoxide angelagert und pro reaktionsfähigem Wasserstoffatom der genannten Amine bis zu 150, vorzugsweise 5 bis 30 Moleküle, Ethylenoxid oder Propylenoxid oder beide angelagert sind. Als Amine oder Alkylolamine sind insbesondere von Interesse: Ethylamin, Propylamin, Isopropylamin, Butylamin, Isobutylamin, Monoethanolamin, Monopropanolamin, Monoisopropanolamin, Monobutanolamin, Monoisobutanolamin, Diethanolamin, Dipropanolamin, Dibutanolamin, Triethanolamin, Tripropanolamin oder Tributanolamin sowie Di- und Polyamine, wie Ethylendiamin, Ethylentriamin, Triethylentetramin, Propylendiamin, Dipropylendiamin, Dipropylentriamin oder Tripropylentetramin.

Bei der Herstellung der erfindungsgemäßen Verbindungen wird, bedingt durch die Vielzahl der Reaktionszentren in den Ausgangsverbindungen, kein reines, einheitliches Endprodukt erhalten, sondern eine Mischung, die jedoch die erfindungsgemäßen Verbindungen als Hauptkomponente, das heißt zu mehr als 50 Gew.-%, vorzugsweise zu mehr als 90 Gew.-%, enthält.

Nach dem erfindungsgemäßen Verfahren werden insbesondere dann Gemische erhalten, wenn ein nach Reaktionsschritt c) erhältliches Oxalkylat in einer einzigen Reaktionsstufe mit mehreren verschiedenen Carbonsäuren verestert wird. Sind bei dieser Veresterung auch Dicarbonsäuren zugegen, so entstehen in der Regel Gemische von verbrückten und nichtverbrückten Oxalkylatestern.

Die Molmasse der erfindungsgemäßen Verbindungen kann in einem weiten Bereich schwanken und reicht von 800 bis 20 000, bevorzugt 2 000 bis 10 000, insbesondere 1000 bis 5000.

Die erfindungsgemäßen Verbindungen sind gelbliche oder grünliche, schaumarme grenzflächenaktive Substanzen, die überraschenderweise für die Herstellung gut fließfähiger und dispersionsstabiler Feststoffdispersionen, insbesondere gut fließfähiger und flockungsstabiler Pigmentpräparationen, geeignet sind.

Die erfindungsgemäßen Verbindungen weisen vielseitige vorteilhafte Eigenschaften auf. Sie gehören zu der Klasse der oberflächenaktiven Verbindungen nach DIN 53900, senken die Oberflächenspannung nach der Ringabreißmethode (DIN 53914) und sind nach den Resultaten im modifizierten Ross-Miles-Test (DIN 53902) als nichtschäumende oder schwachschäumende oberflächenaktive Stoffe zu bezeichnen. Bei geeignetem Hydrophilierungsgrad zeigen sie ausgezeichnetes Netzvermögen für Baumwolle nach der Tauchnetzmethode (DIN 53901) bei gleichzeitig gutem Egalisierverhalten nach DIN 53504. Sie besitzen ein sehr gutes Flockungsschutzvermögen gegenüber Pigmenten und Farbstoffen (DIN 53908) und eine sehr gute Wasserverteilungswirkung als Reinigungsverstärker (DIN 53980) sowie eine gute Auswaschbarkeit als Schmälzmittel (DIN 53504). Die erfindungsgemäßen Verbindungen sind biologisch abbaubar und daher besonders umweltfreundlich.

Die erfindungsgemäßen Verbindungen können aufgrund ihrer vielseitigen oberflächenaktiven Eigenschaften für ein breites Spektrum von Anwendungen eingesetzt werden.

Gegenstand der Erfindung ist deshalb auch die Verwendung einer erfindungsgemäßen Verbindung als oberflächenaktives Mittel. Von besonderem Interesse ist die Verwendung als Dispergier- und Verteilungsmittel für die Feinverteilung und Stabilisierung von anorganischen und organischen Pigmenten, insbesondere von schwerlöslichen oder unlöslichen Farbmitteln, vorzugsweise für die Herstellung von gut fließfähigen Pigmentdispersionen für wäßrige Dispersionsanstrichfarben und für den wäßrigen Druckfarbensektor. Weiterhin von Interesse ist die Verwendung zur Herstellung von Gerbmitteldispersionen auf Metallsalzbasis zum Ein- und Durchfärben von Leder oder zur Herstellung von Dispersionsfarbstoffen, wie sie bevorzugt zum Färben von natürlichen und synthetischen Fasermaterialien, wie Baumwolle, Wolle, Zellulose, Zellwolle, Zelluloseacetat, Zellulosetriacetat, Polyester, Polyamid und Polyacrylnitril, oder von Fasermaterialien, die diese Stoffe enthalten, verwendet werden.

Die erfindungsgemäßen Verbindungen eignen sich außerdem für den Einsatz als Kupplungshilfsmittel und Präparationsmittel bei der Herstellung von Azofarbmitteln, insbesondere von Azopigmenten, sowie als Additive und Emulgatoren, beispielsweise als Korrosionsschutzzusätze, als Zusätze für die Herstellung von Reinigungsverstärkern, Carrier-Emulsionen, Mineralöl- und Fettkörperemulsionen, Metallbearbeitungs- und Korrosionsschutzmitteln sowie von Formulierungen für Pflanzenschutz- und Schädlingsbekämpfungsmitteln. Sie eignen sich ferner als Netz-, Egalisier-, Flotations-, Viskosezusatz-, Viskoseveredlungs- und Färbereihilfsmittel sowie als Broschurhilfsmittel für Textilien und Leder.

Die erfindungsgemäßen Verbindungen können einzeln oder als Gemische sowie in Kombination mit anderen, nichtionogenen sowie gegebenenfalls mit anionaktiven oder kationaktiven Tensiden oder Gemischen davon eingesetzt werden. Weiterhin können Sie zusammen mit üblichen Mengen an Gerüstsubstanzen oder anderen üblichen Zusätzen oder Hilfsstoffen in Emulgier- und Dispergiermittelformulierungen zur Anwendung kommen.

In den folgenden Beispielen beziehen sich "Teile" (TI.) und Prozentangaben auf das Gewicht, Volumenteile verhalten sich zu Gewichtsteilen wie Liter zu Kilogramm. Der Umsetzungsgrad in den jeweiligen Reaktionsstufen wird in den Herstellungsbeispielen durch die Bestimmung der Hydroxyzahl, Säurezahl und Aminzahl charakterisiert. Die Säurezahl (SZ) wird nach DIN 53 402 bestimmt. Die Säurezahl gibt die Menge an Kaliumhydroxid in Milligramm an, die zur Neutralisation von 1 g des Reaktionsprodukts verbraucht wird. Die Hydroxylzahl wird nach DIN 53 240 bestimmt und ist ein Maß für den Gehalt an freien Hydroxylgruppen im Molekül; sie entspricht der Menge an Kaliumhydroxid in mg, welche notwendig ist, die Menge Essigsäure zu neutralisieren, die bei der Acetylierung von 1 g der Prüfsubstanz verbraucht wird. Die Aminzahl wird nach DIN 53 176 bestimmt und ist diejenige Menge an Kaliumhydroxid in Milligramm, die dem Aminanteil von 1 g Substanz äquivalent ist.

### Herstellungsbeispiel 1

### a) Rizinusöl-kolophoniumester

466 Tl. handelsübliches Rizinusöl werden mit 151 Tl. disproportioniertem Kolophonium auf 70 bis 80°C erwärmt und unter Stickstoffgas eine Stunde lang gerührt. Nach Zugabe von 12 Teilen Zinnpulver, 4,0 Teilen p-Toluolsulfonsäure und 150 Vol.-Teilen Xylol wird 16 Stunden lang auf 150 bis 160°C erhitzt und das Reaktionswasser durch Auskreisen entfernt. Nach Abdestillieren des Xylols erhält man ein Produkt mit einer Säurezahl von weniger als 25.

### b) Rizinusöl-kolophoniumester-oxethylat

200 Tl. Rizinusöl-kolophoniumester gemäß Herstellungsbeispiel 1a) werden nach Zugabe von 9 Tl. Natriummethanolat (30 %ig in Methanol) in einem Druckgefäß unter Rühren auf 80°C bis 90°C erhitzt und das Methanol unter reduziertem Druck abgezogen. Nach Erhöhung der Temperatur auf 150°C bis 170°C werden 578 Teile Ethylenoxid bei einem Druck von 4 bis 6 bar aufgepreßt. Danach läßt man noch eine Stunde bei 150°C bis 160°C rühren. Das erhaltene wachsartige braune Rizinusöl-kolophoniumester-oxethylat enthält im Mittel 80 Ethylenoxy-Einheiten pro Molekül und besitzt eine Hydroxyzahl von 27,7.

### Herstellungsbeispiel 2

### Rizinusöl-kolophoniumester-oxethylat-di-sulfobernsteinsäurehalbester

300 Tl. Rizinusöl-kolophoniumester-oxethylat nach Herstellungsbeispiel 1b) werden entsprechend Hydroxylzahl mit 14,5 Tl. Maleinsäureanhydrid bei 70°C bis 80°C in Stickstoffatmosphäre 3 Stunden gerührt. Zu dem erhaltenen Maleinsäurehalbester-Produkt läßt man eine Lösung aus 18,7 Teilen Natriumsulfit in 653 Teilen Wasser zulaufen und zwischen 1 und 2 Stunden bei 70°C bis 80°C so lange rühren, bis die Reaktionsmischung klar wasserlöslich wird. Die Menge des mit der Natriumsulfitlösung zugesetzten Wassers kann 50 bis 85 Gew.-% der das Produkt enthaltenden Lösung betragen. Das erhaltene Hauptprodukt ist ein Sulfobernsteinsäurehalbester, bei dem beide Polyoxyethylenketten bis auf eine Resthydroxylzahl von 4 endständig umgesetzt sind.

### Herstellungsbeispiel 3

### a) Di-kolophonium-rizinusölester

302 Tl. handelsübliches disproportioniertes Kolophonium und 466 Tl. handelsübliches Rizinusöl werden unter Stickstoffgas auf 80 bis 90°C erwärmt. Nach Zugabe von 6 Tl. Zinnpulver und 2 Tl. p-Toluolsulfonsäure und 200 Vol.-Teilen Xylol wird 8 Stunden bei 155 bis 165°C unter Auskreisen des Reaktionswassers verestert. Nach Abdestillieren des Xylols hat das Produkt eine Säurezahl von 6,4.

### b) Di-kolophonium-rizinusölester-oxethylat

300 Tl. Di-kolophonium-rizinusölester nach Herstellungsbeispiel 3a) werden nach Zugabe von 9 Tl. Natriummethanolat (30 %ig in Methanol) analog Herstellungsbeispiel 1b) mit 859,8 Tl. Ethylenoxid umgesetzt. Das erhaltene Esteroxethylat enthält im Mittel 97,7 Ethylenoxid-Einheiten pro Molekül und eine Hydroxylzahl von 9,6.

### Herstellungsbeispiel 4

### Di-kolophonium-rizinusölester-oxethylat-sulfosuccinat

500 Tl. Di-kolophonium-rizinusölester-oxethylat nach Herstellungsbeispiel 3b) werden gemäß Hydroxylzahl mit 8,5 Tl. Maleinsäureanhydrid bei 70 bis 80°C im Stickstoffstrom 3 Stunden gerührt. Zu dem erhaltenen Maleinsäurehalbester-Produkt läßt man eine Lösung aus 10,9 Tl. Natriumsulfit und 964,58 Tl. Wasser zulaufen und rührt 2 bis 3 Stunden bei 70 bis 80°C, bis der Ansatz klar wasserlöslich wird. Die Menge des mit der Natriumsulfitlösung zugesetzten Wassers kann 50 bis 85 Gew.-% des Produktes betragen. Das erhaltene Hauptprodukt ist ein Sulfobernsteinsäurehalbester mit einer Resthydroxylzahl von 0,86.

### Herstellungsbeispiel 5

### a) 3-fach-Rizinusöl-maleinsäureester

200 Tl. handelsübliches hydriertes Rizinusöl werden mit 20,8 Tl. Maleinsäureanhydrid unter Stickstoff innerhalb von 4 Stunden bei 75 bis 85°C verestert. Der erhaltene Rizinusöl-maleinsäurehalbester besitzt eine Säurezahl von 61,2. Nach Zugabe von weiteren 200 Tl. Rizinusöl und 1 Tl. p-Toluolsulfonsäure sowie 200 Vol.-Teilen Xylol wird noch weitere 8 bis 10 Stunden unter Entfernung des Reaktionswassers durch Auskreisen bei 155 bis 165°C bis zu einer Säurezahl von 10 verestert. Nach eine weiteren Zugabe von 220,8 Tl. hydriertem Rizinusölmaleinsäurehalbester wird weitere 5 bis 6 Stunden bei gleicher Temperatur unter Auskreisen des Reaktionswassers verestert. Die gefundene Säurezahl liegt bei 10 bis 12. Das erhaltene Produkt enthält 3 Rizinusöl-Einheiten, die mit 2 Maleinsäure-Einheiten verknüpft sind.

### b) 3-fach-Rizinusöl-maleinsäureester-oxethylat

300 Tl. 3-fach-Rizinusöl-maleinsäureester nach Herstellungsbeispiel 5a) werden nach Zugabe von 9 Tl. Natriummethanolat (30 %ig in Methanol) in einem Druckgefäß unter Rühren auf 80°C erhitzt und das Methanol unter reduziertem Druck abgezogen. Nach Erhöhung der Temperatur auf 150 bis 170°C werden 348,4 Tl. Ethylenoxid bei einem Druck von 4 bis 6 bar aufgepreßt. Danach läßt man noch eine Stunde bei 150°C bis 160°C rühren. Das erhaltene wachsartige braune Oxethylat enthält im Mittel 78,3 Ethylenoxy-Einheiten pro Molekül und besitzt eine Hydroxylzahl von 43,6.

### Herstellungsbeispiel 6

### 3-fach-Rizinusöl-maleinsäureester-oxethylat-benzoat-sulfosuccinat

300 Tl. 3-fach-Rizinusöl-oxethylat nach Herstellungsbeispiel 5b) werden zu 2/5 der Hydroxylzahl mit 28,4 Tl. Benzoesäure bei etwa 20°C gut gemischt und unter Stickstoffatmosphäre nach Zugabe von 1,5 Teilen p-Toluolsulfonsäure und 150 Volumenteilen Xylol auf 150 bis 160°C erhitzt, wobei innerhalb von 8 Stunden das Reaktionswasser durch Auskreisen entfernt wird. Nach Abdestillieren des Schleppmittels und Erreichen einer Säurezahl von etwa 5 wird die Temperatur auf 70 bis 80°C gesenkt und 22,8 Tl. Maleinsäureanhydrid auf 3/5 Anteile der Hydroxylzahl sowie 0,2 Teile Ätznatron zugegeben und weitere 4 Stunden bei gleicher Temperatur gerührt.

Zu dem erhaltenen Maleinsäurehalbester-Produkt läßt man eine Lösung aus 29,4 Teilen Natriumsulfit in 752 Teilen Wasser zulaufen und zwischen 1 und 2 Stunden bei 70°C bis 80°C so lange rühren, bis der Ansatz klar wasserlöslich wird. Die Menge des mit der Natriumsulfitlösung zugesetzten Wassers kann 50 bis 85 Gew.-% der das Produkt enthaltenden Lösung betragen. Das erhaltene Hauptprodukt ist ein Sulfobernsteinsäurehalbester, bei dem alle 5 Polyoxyethylenketten bis auf eine Resthydroxylzahl von 7,3 endständig umgesetzt sind.

### Herstellungsbeispiel 7

### a) 2-fach-Rizinusöl-maleinsäureester

300 Tl. hydriertes Rizinusöl werden mit 32 Tl. Maleinsäureanhydrid 3 Stunden bei 70 bis 80°C gerührt. Nach Zugabe von 3 Tl. p-Toluol-sulfonsäure und 200 Vol.-Teilen Xylol wird 8 Stunden lang auf 135 bis 165°C erhitzt und das Reaktionswasser durch Auskreisen entfernt. Nach Abdestillieren des Xylols hat das Produkt eine Säurezahl von 18. Das erhaltene Produkt enthält 2 Rizinusöl-Einheiten, die durch eine Maleinsäure-Einheit verknüpft sind.

### b) 2-fach-Rizinusöl-maleinsäureester-oxethylat

200 Tl. Rizinusöl-maleinsäureester nach Herstellungsbeispiel 7a) werden nach Zugabe von 1,2 Tl. Natriumhydroxid mit 573,2 Tl. Ethylenoxid, wie in Herstellungsbeispiel 1b) beschrieben, umgesetzt. Das erhaltene wachsartige gelbbraune Rizinusöloxethylat enthält im Mittel 127,4 Ethylenoxid-Einheiten pro Molekül und besitzt eine Hydroxylzahl von 22,3.

### Herstellungsbeispiel 8

### a) Rizinusöl-kolophoniumester-oxalkylat

200 Tl. Rizinusöl-kolophoniumester nach Herstellungsbeispiel 1a) werden vorgelegt und bei 120 bis 140°C und einem Druck von 2 bis 4 bar mit 162 Teilen Propylenoxid umgesetzt. Nach 1 Stunde Nachrührzeit werden bei gleicher Temperatur und gleichen Druckbedingungen nach Zugabe von 6 Teilen Natriummethanolat (30 %ig in Methanol) und Entfernung des Methanols 184,5 Teile Ethylenoxid zugeführt und nochmals 2 Stunden bei 140 bis 145°C und einem Druck von 3 bis 4 bar gerührt. Man erhält ein zähflüssiges grünliches Rizinusölharzester-oxalkylat, das im Mittel 17 Propylenoxid-Einheiten und 25,4 Ethylenoxid-Einheiten pro Molekül enthält. Die Hydroxylzahl des Oxalkylates liegt zwischen 30 und 35.

### b) Rizinusöl-kolophoniumester-oxalkylat-di-maleinsäurehalbester

300 Tl. Rizinusöl-kolophoniumester-oxalkylat nach Herstellungsbeispiel 8a) werden analog Herstellungsbeispiel 2b) gemäß der Hydroxylzahl 32,5 mit 17 Teilen Maleinsäureanhydrid bei 75 bis 80°C innerhalb von 3 bis 4 Stunden verestert. Man erhält ein hellgrünes Halbesterprodukt mit einer Säurezahl von 35.

### c) Triethylentetraminsalz des Rizinusöl-kolophoniumester-oxalkylat-di-maleinsäurehalbesters

300 Tl. Di-maleinsäurehalbester nach Herstellungsbeispiel 8b) werden mit 27,5 Teilen Triethylentetramin innerhalb von 1 bis 1,5 Stunden umgesetzt. Das erhaltene Aminsalz besitzt einen pH-Wert zwischen 8,9 und 9,3 und eine Aminzahl von etwa 125,3.

### Herstellungsbeispiel 9

### a) Rizinusöl-oxethylat

466 Tl. handelsübliches Rizinusöl werden nach Zugabe von 10 Tl. Natriummethanolat (30 % in Methanol) in einem Druckgefäß unter Rühren auf 80°C bis 90°C erhitzt und das Methanol unter reduziertem Druck abgezogen. Nach Erhöhung der Temperatur auf 150 bis 170°C werden 396 Teile Ethylenoxid bei einem Druck von 4 bis 6 bar aufgepreßt. Danach läßt man noch eine Stunde bei 150 bis 160°C rühren. Das erhaltene dickflüssig grüne Rizinusöl-oxethylat enthält im Mittel 18 Ethylenoxy-Einheiten pro Molekül und besitzt eine Hydroxylzahl von 97,6.

### b) 3-fach-Rizinusöl-oxethylat-succinat

200 Tl. Rizinusöl-oxethylat nach Herstellungsbeispiel 9a) werden entsprechend 1/3 der Hydroxylzahl mit 11,6 Tl. Bernsteinsäureanhydrid auf 100 bis 110°C unter Stickstoff erwärmt und gut gemischt. Nach Zugabe von 0,1 Tl. Ätznatron wird bei gleicher Temperatur 4 Stunden gerührt. Entsprechend der erhaltenen Säurezahl werden weitere 206 Tl. Rizinusöl-oxethylat nach Beispiel 9a) zugeführt. Nach Zugabe von 2 Tl. p-Toluolsulfonsäure und 150 Vol.-Teilen Xylol wird 8 Stunden bei 155 bis 165°C verestert, wobei das Reaktionswasser durch Auskreisen am Wasserabscheider entfernt wird. Die Säurezahl beträgt 14,9. Anschließend werden weitere 211,6 Tl. Rizinusöl-oxethylat-bernsteinsäurehalbester zugegeben und weitere 8 Stunden unter Entfernung des Reaktionswassers bei 155 bis 165°C verestert. Danach wird das Xylol abdestilliert und die Säurezahl bestimmt. Das Produkt hat eine Säurezahl von weniger als 10 und eine Hydroxylzahl von 50 bis 60. In dem erhaltenen Produkt sind 3 Rizinusöl-oxethylat-Einheiten durch 2 Bernsteinsäure-Einheiten verknüpft.

### c) 3-fach-Rizinusöl-oxethylat-bernsteinsäureester-oxethylat

400 Tl. Rizinusöl-oxethylat-bernsteinsäureester nach Herstellungsbeispiel 9b) werden nach Zugabe von 18 Tl. Natriummethanolat (30 %ig in Methanol) mit 442 Tl. Ethylenoxid, wie in Herstellungsbeispiel 1a) beschrieben, umgesetzt. Das erhaltene Rizinusölester-oxethylat enthält pro Molekül 192,8 Ethylenoxid-Einheiten bei einer Hydroxylzahl von 22 bis 28.

### Herstellungsbeispiel 10

### a) 2-fach-Rizinusöl-phthalsäureester

466 Tl. handelsübliches Rizinusöl werden nach Zugabe von 37 Tl. Phthalsäureanhydrid, 2 Tl. p-Toluolsulfonsäure und 150 ml Xylol innerhalb von 4 Stunden bei 110°C und 8 Stunden bei 155 bis 165°C verestert und das Reaktionswasser durch Auskreisen entfernt. Nach Abdestillieren des Xylols erhält man ein Produkt mit einer Säurezahl von 12,3. In dem Produkt sind 2 Rizinusöl-Einheiten durch 1 Phthalsäure-Einheit verknüpft.

### b) 2-fach-Rizinsöl-phthalsäureester-oxethylat

200 Tl. 2-fach-Rizinusöl-phthalsäureester nach Herstellungsbeispiel 10a) werden nach Zugabe von 5 Tl. Natriummethanolat (30 %ig in Methanol) mit 176,5 Tl. Ethylenoxid, wie in Herstellungsbeispiel 1b) beschrieben, umgesetzt. Das erhaltene Rizinusölester-oxethylat enthält pro Molekül 40 Ethylenoxid-Einheiten und hat eine Hydroxylzahl von 55 bis 65.

### Herstellungsbeispiel 11

### Rizinusöl-oxethylat-bernsteinsäureester-oxethylat-kolophoniumester-sulfosuccinat

300 Tl. Rizinusöl-oxethylat-succinat-oxethylat nach Herstellungsbeispiel 9c) werden entsprechend 2/5 der Hydroxylzahl mit 25,2 Tl. disproportioniertem Kolophonium unter Stickstoff eine Stunde gerührt. Nach Zugabe von 6 Tl. Zinnpulver, 1 Tl. p-Toluolsulfonsäure und 150 Vol.-Teilen Xylol wird 10 Stunden unter Auskreisen des Reaktionswassers verestert. Nach Abdestillieren des Xylols beträgt die Säurezahl weniger als 20. Anschließend werden entsprechend 3/5 der Hydroxylzahl 8,2 Tl. Maleinsäureanhydrid eingetragen. Es wird 3 Stunden bei 75 bis 80°C halbverestert und durch Zugabe einer Lösung aus 10,6 Tl. Natriumsulfit in 639 Tl. Wasser zum Sulfosuccinat umgesetzt. Die Menge des zugesetzten Wassers liegt vorzugsweise zwischen 50 und 85 % der fertigen Produktlösung. Das erhaltene Produkt ist ein Sulfobernsteinsäurehalbester mit einer Resthydroxylzahl von 3,3, bei dem alle 5 Polyoxyethylenketten endständig umgesetzt sind.

### Herstellungsbeispiel 12

### a) 2-fach-Rizinusöl-dimerfettsäureester

400 Tl. handelsübliches Rizinusöl werden mit 121 Tl. handelsüblicher Dimerfettsäure (®Pripol 1022) nach Zugabe von 2 Tl. p-Toluolsulfonsäure und 150 Vol.-Teilen Xylol innerhalb von 8 Stunden bei 155 bis 165°C verestert, wobei das Reaktionswasser durch Auskreisen am Wasserabscheider entfernt wird. Danach wird das Xylol abdestilliert und man erreicht eine Säurezahl von 6,0. Das erhaltene Produkt enthält 2 Rizinusöl-Einheiten, die durch eine Dimerfettsäure-Einheit verknüpft ist.

### b) 2-fach-Rizinusöl-dimerfettsäureester-oxethylat

300 Tl. 2-fach-Rizinusöl-dimerfettsäureester nach Herstellungsbeispiel 1a) werden nach Zugabe von 2,5 Tl. pulverisiertem Ätznatron mit 529 Tl. Ethylenoxid analog Herstellungsbeispiel 1a) umgesetzt. Das erhaltene Produkt enthält im Mittel 60 Ethylenoxid-Einheiten pro Molekül bei einer Hydroxylzahl von 50 bis 60.

### Herstellungsbeispiel 13

### a) 2-fach-Rizinusöl-dimerfettsäureester-oxethylat-tetra-sulfosuccinat

300 Tl. 2-fach-Rininusöl-dimerfettsäureester-oxethylat nach Herstellungsbeispiel 12b) werden entsprechend Hydroxylzahl mit 28,8 Tl. Maleinsäureanhydrid gemischt und unter Stickstoff 3 Stunden bei 70 bis 80°C gerührt. Zu dem erhaltenen Maleinsäurehalbester läßt man eine Lösung aus 37,0 Tl. Natriumsulfit in 679,3 Vol.-Teilen Wasser zulaufen und 2 bis 3 Stunden bei 70 bis 80°C so lange rühren, bis der Ansatz klar wasserlöslich ist. Die Menge des mit der Natriumsulfitlösung zugesetzten Wassers kann 50 bis 85 Gew.-% der das Produkt enthaltenden Lösung betragen. Das erhaltene Hauptprodukt ist ein Sulfobernsteinsäurehalbester mit einer Resthydroxylzahl von 8,1, bei dem alle 4 Polyoxyethylenketten endständig umgesetzt sind.

### Herstellungsbeispiel 14

### a) Rizinolsäure-kolophonium-glyzerinmischester

298 Tl. handelsübliche Rizinolsäure werden mit 302 Tl. disproportioniertem Kolophonium auf 70 bis 80°C erwärmt und unter Stickstoffgas eine Stunde lang gerührt. Nach Zugabe von 12 Teilen Zinnpulver, 4,0 Teilen p-Toluolsulfonsäure und 150 Vol.-Teilen Xylol wird 16 Stunden lang auf 150 bis 160°C erhitzt und das Reaktionswasser durch Auskreisen entfernt. Man erhält eine Säurezahl von weniger als 25. Anschließend werden 92 Tl. Glyzerin zugegeben und weitere 8 Stunden bei 155 bis 160°C unter Entfernung des Reaktionswassers bis zu einer Säurezahl von weniger als 18 weiterverestert und anschließend das Xylol abdestilliert.

### b) Rizinolsäure-mischester-oxalkylat

300 Tl. Rizinolsäure-mischester nach Herstellungsbeispiel 14a) werden vorgelegt und bei 120 bis 140°C und einem Druck von 3 bis 4 bar mit 268 Teilen Propylenoxid umgesetzt. Nach 1 Stunde Rühren werden bei gleicher Temperatur und gleichen Druckbedingungen nach Zugabe von 6 Teilen Natriummethanolat (30 %ig in Methanol) und Entfernung des Methanols 305 Teile Ethylenoxid zugeführt und nochmals 1 Stunde bei 140 bis 145°C und einem Druck von 3 bis 4 bar gerührt. Man erhält ein dickflüssiges grünliches Rizinolsäureester-Oxalkylat, das im Mittel 10 Propylenoxid-Einheiten und 16 Ethylenoxid-Einheiten pro Molekül enthält. Die Hydroxylzahl des Oxalkylates liegt zwischen 110 und 120.

### Herstellungsbeispiel 15

### a) Rizinolsäure-mischester-oxalkylat-dimaleinsäurehalbester

300 Tl. Rizinolsäure-mischester-oxalkylat nach Herstellungsbeispiel 14b) werden gemäß einer Hydroxylzahl von 115 mit 60,3 Tl. Maleinsäureanhydrid bei 75 bis 80°C innerhalb von 3 bis 4 Stunden halbverestert. Man erhält ein gelbgrünes Halbesterprodukt mit einer Säurezahl von 95.

### b) Diethylentriaminsalz

300 Tl. des Di-maleinsäurehalbesters nach Herstellungsbeispiel 15a) werden entsprechend Säurezahl mit 52,8 Tl. Diethylentriamin innerhalb von 1 bis 2 Stunden bei 60 bis 70°C umgesetzt. Man erhäft ein gelbgrünes, wachsartiges Produkt mit einer Aminzahl zwischen 117 und 122.

### Herstellungsbeispiel 16

### a) Rizinolsäure-mischester-oxethylat

300 Tl. Rizinolsäure-mischester nach Herstellungsbeispiel 14a) werden nach Zugabe von 3 Tl. Natriummethanolat (30 % in Methanol) in einem Druckgefäß unter Rühren auf 80 bis 90°C erhitzt, und unter reduziertem Druck wird das Methanol abgezogen. Nach Erhöhung der Temperatur auf 150 bis 170°C werden 360 Teile Ethylenoxid bei einem Druck von 4 bis 6 bar aufgepreßt. Danach läßt man noch eine Stunde bei 150 bis 160°C rühren. Das erhaltene wachsartige grüne Rizinolsäureoxethylat enthält im Mittel 28 Ethylenoxy-Einheiten pro Molekül und besitzt eine Hydroxylzahl von 180 bis 190.

### b) Rizinusöl-mischester-oxethylat-di-sulfosuccinat

200 Tl. Rizinusöl-mischester-oxethylat nach Herstellungsbeispiel 14b) werden in Gegenwart von 0,1 Tl. pulverisiertem Ätznatron entsprechend Hydroxylzahl mit 64,7 Tl. Maleinsäureanhydrid innerhalb von 4 Stunden bei 70 bis 80°C verestert und anschließend durch Zugabe einer Lösung von 83,2 Teilen Natriumsulfit in 668 Teilen Wasser zum Sulfosuccinat umgesetzt. Die Menge des zugesetzten Wassers kann zwischen 50 und 85 Gew.-% der das Produkt enthaltenden Lösung betragen. Das erhaltene Hauptprodukt ist ein Sulfobernsteinsäurehalbester mit einer Resthydroxylzahl von 24,3, bei dem beide Polyoxyethylenketten endständig umgesetzt sind.

### Herstellungsbeispiel 17

### a) 2-fach-Rizinolsäure-phthalsäureester

298 Tl. handelsübliche Rizinolsäure werden mit 74 Tl. Phthalsäureanhydrid in Gegenwart von 0,4 Tl. pulverisiertem Ätznatron innerhalb 10 Stunden bei 100 bis 110°C analog Herstellungsbeispiel 10a) bis zu einer Säurezahl von 16 verestert. Nach Zugabe von 149 Tl. Triethanolamin wird nochmals 8 Stunden lang bei 155 bis 160°C bei gleichzeitigem Auskreisen des Reaktionswassers verestert. Nach Abdestillieren des Xylols erhält man ein Produkt mit einer Säurezahl kleiner als 18.

### b) 2-fach-Rizinolsäure-phthalsäure-mischester-oxethylat

300 Tl. 2-fach-Rizinolsäure-phthalsäure-mischester nach Herstellungsbeispiel 17a) werden nach Zugabe von 4,5 Tl. Natriummethanolat (30 %ig in Methanol) in einem Druckgefäß unter Rühren auf 80 bis 90°C erhitzt, und unter reduziertem Druck wird das Methanol abgezogen. Nach Erhöhung der Temperatur auf 150 bis 170°C werden 860 Teile Ethylenoxid bei einem Druck von 4 bis 6 bar aufgepreßt. Danach läßt man noch eine Stunde bei 150 bis 160°C rühren. Das erhaltene zähflüssige grünbraune Oxethylat enthält im Mittel 58 Ethylenoxy-Einheiten pro Molekül und besitzt eine Hydroxylzahl von 60 bis 70.

### Herstellungsbeispiel 18

### 2-fach-Rizinolsäure-phthalsäure-mischester-oxethylat-disulfosuccinat

300 Tl. des 2-fach-Rizinolsäuremischester-oxethylats nach Herstellungsbeispiel 17b) werden entsprechend Hydroxylzahl mit 34,1 Tl. Maleinsäureanhydrid in 3 Stunden bei 70 bis 80 °C halbverestert und mit 43,4 Tl. Natriumsulfit, gelöst in 700 Tl. Wasser, bei gleicher Temperatur in 2 bis 3 Stunden bis zur Löslichkeit umgesetzt.

Das erhaltene Hauptprodukt ist ein Sulfobernsteinsäurehalbester mit einer Resthydroxylzahl von 7,0, bei dem alle 4 Polyoxyethylenketten endständig umgesetzt sind.

### Herstellungsbeispiel 19

### a) 2-fach-Rizinolsäure-succinat-diglycerin-ester

298 Tl. handelsübliche Rizinolsäure werden mit 50 Tl. Bernsteinsäureanhydrid bei 70 bis 80°C unter Stickstoffgas gemischt. Nach Zugabe von 1,5 Tl. p-Toluolsulfonsäure und 200 Vol.-Teilen Xylol wird 3 Stunden bei 100 bis 110°C und anschließend 10 Stunden bei 155 bis 165°C verestert, wobei das Reaktionswasser durch Auskreisen entfernt wird. Nach Vorliegen einer Säurezahl von weniger als 20 werden 92 Tl. Glyzerin zugegeben und weitere 8 Stunden bei 155 bis 165°C bei nochmaliger Entfernung des Reaktionswassers weiterverestert. Nach Abdestillieren des Xylols hat das Produkt eine Säurezahl von 5.

### b) 2-fach-Rizinolsäure-succinat-diglycerin-ester-oxethylat

200 Tl. des Rizinolsäuremischesters nach Herstellungsbeispiel 19a) werden nach Zugabe von 6 Tl. Natriummethanolat (30 % in Methanol) mit 573 Tl. Ethylenoxid analog Herstellungsbeispiel 1b) umgesetzt. Das erhaltene Produkt enthält als Hauptbestandteil ein Dirizinolsäure-succinat-diglyzerinester-oxethylat mit im Mittel 55 Ethylenoxid-Einheiten pro Molekül und einer Hydroxylzahl von 65 bis 75.

### Herstellungsbeispiel 20

### a) 2-fach-Rizinolsäure-succinat-diglycerin-oxethylat-tetra-phthalsäurehalbester

300 Tl. des Rizinolsäure-mischester-oxethylats entsprechend Herstellungsbeispiel 19b) werden nach Hydroxylzahl mit 55,4 Tl. Phthalsäureanhydrid bei Überlagerung von Stickstoffgas bei 70 bis 80°C gemischt und nach Zugabe von 0,2 Tl. pulverisiertem Ätznatron innerhalb von 4 Stunden bei 100 bis 110°C verestert. Man erhält im Mittel pro Molekül einen 4-fachen Phthalsäurehalbester mit einer Säurezahl von 59.

### b) 4-faches Diethylentriaminsalz

300 Tl. des Phthalsäurehalbesters nach Herstellungsbeispiel 20a) werden entsprechend Säurezahl mit 31,5 Tl. Diethylentriamin bei 70 bis 80°C umgesetzt. Das erhaltene Aminprodukt besitzt einen pH-Wert zwischen 8,5 und 9,5 und eine Aminzahl von etwa 136.

### Herstellungsbeispiel 21

### 2-fach-Rizinolsäure-succinat-diglycerin-oxethylat-tetra-sulfosuccinat

300 Tl. des Rizinolsäure-mischester-oxethylats entsprechend Herstellungsbeispiel 19b) werden nach Hydroxylzahl mit 37 Tl. Maleinsäureanhydrid unter Stickstoffgas bei 70 bis 80°C in 3,5 Stunden halbverestert und anschließend bei gleicher Temperatur mit einer Lösung aus 47,6 Tl. Natriumsulfit und 714 Tl. Wasser in 2 bis 3 Stunden bis zum Klarwerden des Ansatzes umgesetzt. Die Menge des zugesetzten Wassers kann zwischen 50 und 85 % der das Produkt enthaltenden Lösung betragen. Das erhaltene Hauptprodukt ist ein Sulfobernsteinsäurehalbester mit einer Resthydroxylzahl von 7,6, bei dem alle 4 Polyoxyethylenketten endständig umgesetzt sind.

### Herstellungsbeispiel 24

### a) 3-fach-Rizinusöl-maleinsäureester-oxethylat

300 Tl. 3-fach-Rizinusöl-maleinsäureester nach Herstellungsbeispiel 5a) werden nach Zugabe von 9 Teilen Natriummethanolat (30 %ig in Methanol) in einem Druckgefäß unter Rühren auf 80 bis 90°C erhitzt. Unter reduziertem Druck wird das Methanol abgezogen. Nach Erhöhung der Temperatur auf 150 bis 170°C werden 860 Tl. Ethylenoxid bei einem Druck von 4 bis 6 bar aufgepreßt. Danach läßt man noch eine Stunde bei 150 bis 160°C rühren. Das erhaltene wachsartige grünbraune Oxethylat enthält im Mittel 19,5 Ethylenoxy-Einheiten pro Molekül und besitzt eine Hydroxylzahl von 24,5.

### b) 3-fach-Rizinusöl-penta-maleinsäurehalbester-1,3-propandiamin-Salz

400 Tl. 3-fach-Rizinusöl-oxethylat nach Herstellungsbeispiel 24a) werden nach der vorliegenden Hydroxylzahl mit 17 Tl. Maleinsäureanhydrid nach Zugabe von 0,2 Tl. gepulvertem Ätznatron 4 Stunden bei 70 bis 80°C halbverestert. Nach Abkühlung auf 50 bis 55°C werden entsprechend einer Säurezahl von 23,3 innerhalb von 30 bis 60 Minuten 12,3 Tl. 1,3-Propandiamin, gelöst in 430 Tl. Wasser, zugetropft und danach 2 Stunden gerührt. Das vorliegende Aminsalz besitzt einen pH-Wert von 8,1 bis 8,5 und eine Aminzahl von etwa 40 bis 50.

### Herstellungsbeispiel 25

### 3-fach-Rizinusöl-penta-maleinsäurehalbester-1,3-propandiamin-oxethylat-Salz

300 Tl. 3-fach-Rizinusöl-maleinsäureester-oxethylat nach Herstellungsbeispiel 5b) werden nach vorliegender Hydroxylzahl mit 22,9 Tl. Maleinsäureanhydrid bei 70 bis 80°C unter Zugabe von 0,1 Tl. gepulvertem Ätznatron in 4 Stunden halbverestert. Nach Abkühlung auf 50 bis 60°C werden entsprechend einer Säurezahl von 44,1 innerhalb von 60 bis 120 Minuten 375 Tl. 1,3-Propandiamin, welches mit 32 Ethylenoxid-Einheiten oxethyliert ist, zugetropft und danach 2 Stunden bei 20 bis 25°C gerührt. Das vorliegende Aminsalzaddukt besitzt einen pH-Wert von 7,5 bis 8 und eine Aminzahl von etwa 60 bis 70.

### Herstellungsbeispiel 26

### 2-fach-Rizinusöl-tetra-sulfobernsteinsäurehalbester-1,3-propandiamin-oxethylat-Salz

300 Tl. des 2-fach-Rizinusöl-maleinsäureester-oxethylats nach Herstellungsbeispiel 7b) werden entsprechend Hydroxylzahl nach Zugabe von 0,1 Tl. gepulvertem Ätznatron mit 11,7 Tl. Maleinsäureanhydrid bei 70 bis 80°C innerhalb von 3 Stunden halbverestert. Nach Zulauf einer Lösung aus 15 Teilen Natriumhydrogensulfit und 608,0 Teilen Wasser wird weitere 2 Stunden bei 70 bis 80°C gerührt. Nach Absinken der Temperatur auf 20 bis 25°C werden entsprechend einer Säurezahl von 26,3 innerhalb von 30 bis 60 Minuten 226 Teile 1,3-Propandiamin, welches mit 32 Mol Ethylenoxid oxethyliert ist, zugetropft und 2 Stunden bei 20 bis 25°C gerührt. Das vorliegende Aminsalz besitzt einen pH-Wert von 7,3 bis 8,0 und eine Aminzahl von etwa 45.

### Herstellungsbeispiel 29

### Rizinusöl-harzester-oxethylat-di-schwefelsäurehalbester

300 Tl. des Rizinusöl-harzester-oxethylats nach Herstellungsbeispiel 1b) werden entsprechend Hydroxylzahl mit 14,5 Tl. Amidosulfonsäure und 3,8 Tl. Harnstoff vermischt, 4 Stunden bei 122 bis 125°C unter Stickstoffatmosphäre gerührt, wobei das sehr zähe Reaktionsprodukt (Ammoniumsalz des Bis-schwefelsäurehalbesters des mit 80 Ethylenoxid-Einheiten oxethylierten Kolophonium-Rizinusölesters) wasserlöslich wird. Zur Überführung in das Natriumsalz werden 300 Tl. des Ammoniumsalzes mit 860 Tl. Wasser und 40 ml 1-normaler Natronlauge versetzt und unter Einleiten von Stickstoff und Rühren so lange auf 70°C erwärmt, bis der Ammoniak ausgetrieben ist. Die Menge des zugesetzten Wassers kann 50 bis 85 Gew.-% der das Produkt enthaltenden Lösung betragen. Das erhaltene Hauptprodukt ist ein Schwefelsäurehalbester mit einer Resthydroxylzahl von 5,5, bei dem alle 2 Polyoxyethylenketten endständig umgesetzt sind.

### Herstellungsbeispiel 35

### 2-fach Rizinusöl-maleinsäureester-oxethylat-tetra-ölsäureester

300 Tl. Rizinusöl-oxethylat nach Herstellungsbeispiel 7b) werden gemäß Hydroxylzahl mit 33,6 Tl. handelsüblicher Ölsäure unter Stickstoffgas bei 70 bis 80°C eine Stunde lang gerührt. Nach Zugabe von 2 Tl. p-Toluolsulfonsäure und 150 Vol.-Teilen Xylol wird 18 Stunden lang auf 153 bis 165°C erhitzt und das Reaktionswasser durch Auskreisen entfernt. Nach Abdestillieren des Xylols erhält man ein Produkt mit einer Säurezahl von weniger als 20, bei dem alle 4 Polyoxethylenketten endständig umgesetzt sind.

### Vergleichsbeispiel 1

11 Tl. Dinitroanilin werden in üblicher Weise in einer Mischung aus Schwefelsäure und Salzsäure mit 10,4 Teilen 40 %iger wäßriger Natriumnitritlösung diazotiert.

Aus einer Lösung von 8,64 Teilen β-Naphthol in einer Mischung von 100 Teilen Wasser und 8 Teilen 33 %iger Natronlauge wird reines β-Naphthol gefällt, indem man die Lösung zu einer Mischung von 300 Teilen Wasser und 10 Teilen 31 %iger Salzsäure tropft.

Die Kupplung zum C.I. Pigment Orange 5 (C.I. No. 12075) erfolgt sodann in üblicher Weise durch Zulauf der geklärten Diazoniumsalzlösung zur Suspension des gefällten β-Naphthols. Anschließend wird das Pigment filtriert, mit Wasser gewaschen und schließlich getrocknet.

### Vergleichsbeispiel 2

34,5 Teile 4-Chlor-2-nitroanilin werden in verdünnter Salzsäure angerührt, dann mit 34,7 Teilen 40 %iger Natriumnitritlösung in üblicher Weise diazotiert und anschließend geklärt.

In ein Gemisch aus 800 Teilen Wasser, 4 Teilen Eisessig und 8,1 Teilen 33 %iger Natronlauge werden 43,25 Teile Acetessigsäure-2-chloranilid eingerührt. Die Kupplung zum C.I. Pigment Yellow 3 (C.I. No. 11710) erfolgt bei 10 bis 20°C derart, daß die Diazoniumsalzlösung unter die Oberfläche der Kupplungssuspension zuläuft. Der pH-Wert wird dabei durch Zugabe von verdünnter Natronlauge bei pH 4 bis 4,5 gehalten. Anschließend wird das Pigment filtriert, mit Wasser gewaschen und schließlich getrocknet.

### Anwendungsbeispiel 3

60,2 Teile 1-Acetoacetylamino-2,4-dimethylbenzol und 6,1 Teile 1-Acetoacetylamino-2,5-dimethoxy-4-chlorbenzol werden in 900 Teilen Wasser und 31 Volumenteilen 33 %iger Natronlauge gelöst und nach Zusatz von 1,5 Teilen eines Fettalkoholpolyglykolethers mit Hilfe von 22 Volumenteilen Essigsäure gefällt. Nach Zugabe von 2,5 Teilen des Produkts aus Herstellungsbeispiel 8 kuppelt man mit einer Lösung von tetrazotiertem 4,4'-Diamino-3,3'-dichlor-diphenyl, wobei die Tetrazoniumsalzlösung durch Zugabe von 60 Volumenteilen wäßriger 5-normaler Natriumnitritlösung zu einem Gemisch aus 38 Teilen 4,4'-Diamino-3,3'-dichlordiphenyl, 183 Volumenteilen 5-normaler Salzsäure und 520 Teilen Wasser hergestellt wird. Nach beendeter Kupplung wird die Pigmentsuspension mit 2,5 Teilen Dehydroabietylamin versetzt, alkalisch gestellt, anschließend mit einer Lösung, die 1,8 Teile Dimethylcocosfettaminoxid und 36 Teile eines partiell hydrierten Kolophoniums enthält, versetzt und 30 Minuten bei 98°C erhitzt. Danach stellt man mit Salzsäure auf pH 4 und erhitzt weitere 30 Minuten bei 98°C. Anschließend wird filtriert, gewaschen und getrocknet. Man erhält eine Pigmentpräparation, die beim Einarbeiten in einen Druckfarbenfirnis für den Buch- und Offsetdruck eine Druckfarbe mit sehr guten anwendungstechnischen Eigenschaften ergibt. Die Druckfarbe zeichnet sich im Vergleich zu einer Druckfarbe, die ohne den Zusatz des Produkts aus dem Herstellungsbeispiel 15 hergestellt worden ist, durch ein deutlich verbessertes Fließverhalten aus.

Gleich gute Ergebnisse erhält man bei Einsatz der erfindungsgemäßen Verbindungen aus den Herstellungsbeispielen 8c), 20b).

### Anwendungsbeispiel 4

1012 Teile 3,3'-Dichlor-4,4'-diamino-diphenyl werden mit 6000 Teilen Wasser und 2500 Volumenteilen 30%iger Salzsäure angerührt und anschließend mit 1052 Volumenteilen 40 %iger Natriumnitritlösung bei 0 bis 15°C bis-diazotiert. Zur Vorbereitung der Kupplungsreaktion werden 1466 Teile Acetoacetylaminobenzol in 10 000 Teilen Wasser und 800 Volumenteilen 33 %iger Natronlauge gelöst, mit 160 Teilen des nach Herstellungsbeispiel 11 hergestellten Tensids versetzt und durch Zugabe von 700 Volumenteilen 80 %iger Essigsäure gefällt. Die Azokupplung erfolgt unter langsamem Zulaufen der hergestellten Lösung des Bis-diazoniumsalzes zur Suspension der gefällten Kupplungskomponente, wobei der pH-Wert durch laufende Zugabe von 6 %iger Natronlauge bei etwa 4,5 gehalten wird. Nach Beendigung der Kupplung wird die essigsaure Suspension auf 50°C erwärmt und mit 150 Teilen Dodecyl-benzyl-dimethyl-ammoniumchlorid, 750 Teilen Talgfettpropylendiamin und 300 Teilen Bis-(4-aminocyclohexyl)-methan versetzt. Man erhitzt auf 90 bis 100°C und hält diese Temperatur eine halbe Stunde lang aufrecht. Dann wird die Mischung mit 1500 Volumenteilen 33 %iger Natronlauge alkalisch gestellt und einige Stunden bei 90 bis 100°C gerührt. Anschließend wird das Produkt filtriert, gewaschen, getrocknet und gemahlen. Man erhält eine Präparation von C.I. Pigment Yellow 12 (C.I. No. 21090) die sich gut zum Pigmentieren von Tiefdruckfarben auf Toluolbasis verwenden läßt. Die erhaltene Pigmentpräparation von Pigment Yellow 12 zeigt sehr gute Resultate bezüglich Farbstärke, Glanz, Transparenz und Gradationsverhalten. Die damit pigmentierten Tiefdruckfarben zeichnen sich auch durch eine vorteilhaft niedrige Viskosität aus.

Ebenso gute Ergebnisse erhält man bei Einsatz der erfindungsgemäßen Verbindungen aus den Herstellungsbeispielen 2), 4), 16b).

### Anwendungsbeispiel 5

In einem Mahlbehälter mit 1 l Inhalt werden 68 Teile Propandiol-1,2, 24 Teile Harnstoff, 60 Teile Wasser, 0,8 Teile Konservierungsmittel sowie 28 Teile des Produkts aus Herstellungsbeispiel 1 vorgelegt und homogenisiert.

Nachdem am Dissolver 200 Teile C.I. Pigment Green 7 (C.I. No. 74260) eingerührt worden sind, fügt man 1100 Teile Siliquarzit-Perlen von 1 mm Durchmesser zu und läßt 1 Stunde in einer diskontinuierlichen Rührwerkskugelmühle mahlen. Das Mahlgut wird anschließend mit weiteren 19,2 Teilen Wasser verdünnt, von den Mahlkörpern getrennt und entlüftet. Man erhält eine lagerstabile, gut fließfähige Pigmentdispersion mit einem Pigmentgehalt von 50 %. Die Pigmentpräparation zeigt in verschiedenen Dispersionsanstrichfarben einwandfreie Flockungsstabilität.

Gleich gute Ergebnisse erhält man bei Einsatz der erfindungsgemäßen Verbindungen aus den Herstellungsbeispielen 3b), 7b).

### Anwendungsbeispiel 6

Analog Anwendungsbeispiel 5 werden 188 Teile C.I. Pigment Blue 15:3 (C.I. No. 74160) in einer Mischung aus 76 Teilen Propandiol-1,2, 0,8 Teilen Konservierungsmittel, 16 Teilen Harnstoff, 70 Teilen Wasser und 30 Teilen des Produkts aus Herstellungsbeispiel 9 in einer Perlmühle dispergiert.

Nach Verdünnen mit 19,2 Teilen Wasser erhält man eine lagerstabile, gut fließfähige Pigmentdispersion mit einem Pigmentgehalt von 47 %. Neben einwandfreier Flockungsstabilität in verschiedenen Dispersionsanstrichfarben eignet sich die Präparation hervorragend zum Pigmentieren von Textildruckpasten.

Ähnlich gute Ergebnisse erhält man bei Einsatz der erfindungsgemäßen Verbindungen aus den Herstellungsbeispielen 1), 3b).

### Anwendungsbeispiel 7

Analog Anwendungsbeispiel 5 werden 160 Teile C.I. Pigment Red 146 (C.I. No. 12 485) in einer Mischung aus 76 Teilen Propandiol-1,2, 0,8 Teilen Konservierungsmittel, 20 Teilen Harnstoff, 72 Teilen Wasser und 32 Teilen des Produkts aus Herstellungsbeispiel 22b) in einer Perlmühle dispergiert.

Nach Verdünnen mit 39,2 Teilen Wasser erhält man eine lagerstabile, gut fließfähige Pigmentdispersion mit einem Pigmentgehalt von 40 %. Aufgrund der sehr guten Flockungsstabilität eignet sich die Präparation zum Pigmentieren von wäßrigen Dispersionsanstrichfarben, wäßrig/alkoholischen Flexodruckfarben und Textildruckfarben.

Ähnlich gute Ergebnisse erhält man bei Einsatz der erfindungsgemäßen Verbindungen aus den Herstellungsbeispielen 5), 6).

### Anwendungsbeispiel 8

Analog Anwendungsbeispiel 5 werden 140 Teile C.I. Pigment Yellow 83 (C.I. No. 21108) in einer Mischung aus 60 Teilen Propandiol-1,2, 0,8 Teilen Konservierungsmittel, 53 Teilen Wasser und 114 Teilen einer 35 %igen wäßrigen Lösung des in Herstellungsbeispiel 6 beschriebenen Produkts in einer Perlmühle dispergiert. Nach Verdünnen mit 32,2 Teilen Wasser erhält man eine lagerstabile, gut fließfähige Pigmentdispersion mit einem Pigmentgehalt von 35 %.

Die erhaltene Pigmentdispersion eignet sich hervorragend für die Papiermasse-Einfärbung und zeichnet sich hierbei besonders durch geringe Schaumneigung aus.

Ebenso deutlich verbesserte Ergebnisse erhält man bei Einsatz der erfindungsgemäßen Verbindungen aus den Herstellungsbeispielen 11), 21), 27b) oder 34).

### Anwendungsbeispiel 9

138 Teile C.I. Pigment Brown (C.I. No. 12480) werden mit 127 Teilen einer 35%igen wäßrigen Lösung nach Herstellungsbeispiel 6 und 134 Teilen Wasser in einer Rührwerkskugelmühle mit Siliquarzit-Perlen (1 mm Durchmesser) gemahlen und anschließend durch Zugabe von 129 Teilen Wasser verdünnt. Die auf diese Weise erhaltene sehr gut fließfähige Pigmentdispersion eignet sich hervorragend zum Ein- und Durchfärben von Leder, für die Einstellung von Tief- und Flexodruckfarben sowie für die Papiermassefärbung.

### Anwendungsbeispiel 10

99 Teile C.I. Sulfur Brown 16 (C.I. No. 53 285) werden mit 229 Teilen einer 35 %igen wäßrigen Lösung nach Herstellungsbeispiel 6) in einer Rührwerkskugelmühle gemahlen und anschließend durch Zugabe von 58 Teilen Wasser verdünnt. Die erhaltene Pigmentdispersion eignet sich besonders für das Einfärben von Leder nach dem Ausziehverfahren sowie gleichermaßen für die Aufziehfärbung.

### Anwendungsbeispiel 11

50 Teile C.I. Disperse Orange 13 (C.I. No. 26 080) werden mit 85,7 Teilen einer 35 %igen wäßrigen Lösung nach Herstellungsbeispiel 6 und 110 Teilen Wasser in einer Rührwerksmühle 4 Stunden bis zur Feinverteilung gemahlen. Nach Zugabe von 50 Teilen Wasser erhält man einen 20 %igen Farbteig mit sehr guter Lagerstabilität und sehr gutem Fließverhalten, der sich zum Färben von Polyester-, Polyester/Wolle- und Polyester/Zellwolle-Mischgespinsten eignet.

### Anwendungsbeispiel 12

75 Teile C.I. Pigment Black 7 (C.I. No. 77266) werden mit 49 Teilen einer 50 %igen wäßrigen Lösung nach Herstellungsbeispiel 8 und 80 Teilen Wasser entsprechend Anwendungsbeispiel 4 gemahlen und mit weiteren 13 Teilen Wasser verdünnt. Die erhaltene Rußdispersion besitzt ein gutes Fließverhalten und eignet sich hervorragend zum Ein- und Durchfärben von Leder nach dem Bad- und Tauchverfahren.

### Anwendungsbeispiel 13

137 Teile Zirconiumcarbonat werden mit 127 Teilen einer 35 %igen wäßrigen Lösung nach Herstellungsbeispiel 6 und 100 Teilen Wasser in einer Rührwerkskugelmühle mit Siliquarzit-Perlen (1 mm Durchmesser) gemahlen und mit weiteren 37 Teilen Wasser versetzt. Die lagerstabile Zirconiumcarbonat-Dispersion eignet sich besonders zum Gerben von Leder.

### Anwendungsbeispiel 14

50 Teile Titandioxid werden mit 46 Teilen einer 35 %igen wäßrigen Lösung nach Herstellungsbeispiel 26 und 48,5 Teilen Wasser analog Anwendungsbeispiel 12 gemahlen und feinverteilt. Die erhaltene Titandioxid-Suspension ist sehr stabil und zum Färben von Leder nach den üblichen Methoden sehr gut geeignet.

### Anwendungsbeispiel 15

75 Teile Methylnaphthalin werden mit 25 Teilen des Diethylentriaminsalzes nach Herstellungsbeispiel 6 homogen verrührt. Das Konzentrat wird anschließend mit Wasser auf 1000 Volumenteile aufgefüllt. Man erhält eine feindisperse Carrier-Emulsion, die bei der verwendeten üblichen Verdünnung (1:10) eine hervorragende Stabilität besitzt und über längere Zeit verwendet werden kann.

### Anwendungsbeispiel 16

70 Teile eines aromatenhaltigen Mineralöls werden mit 20 Teilen des Diethylentriaminsalzes nach Herstellungsbeispiel 15 und 10 Teilen des Oxethylates nach Herstellungsbeispiel 12 homogen verrührt und anschließend mit Wasser auf 900 Volumenteile verdünnt. Man erhält eine feindisperse bis transparente Mineralöl-Emulsion, die nach einer Verdünnung von 1:9 neben einer hervorragenden Stabilität über längere Zeit zusätzlich Korrosionsschutzeigenschaften nach DIN 51 360 besitzt.

### Anwendungsbeispiel 17

50 Teile des Pflanzenschutzmittels 2-Carbomethoxy-amino-benzimidazol werden mit 68 Teilen einer 35 %igen wäßrigen Lösung nach Herstellungsbeispiel 6 und 82 Teilen Wasser bis zur Feinverteilung in einer 1-Liter-Rührwerksmühle gemahlen. Nach Abtrennung der Mahlkörper erhält man eine sehr stabile Dispersion mit einer guten Schwebefähigkeit ohne Bodensatz. Gleich gute Ergebnisse erhält man bei Einsatz der erfindungsgemäßen Verbindungen aus den Herstellungsbeispielen 13), 16b).

## Patentansprüche

1. Verbindung auf der Basis eines Rizinusöles oder einer Rizinolsäure, bestehend aus
a) 1 bis 10 Einheiten der Formel (la) 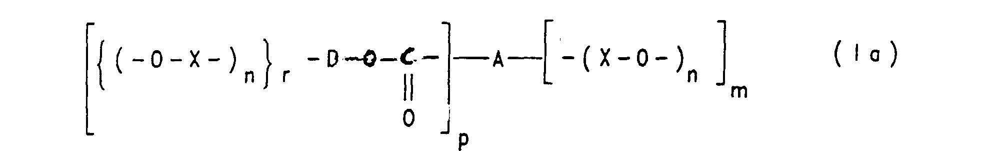
b) 1 bis 72 monovalenten Resten der Formel (Ib)
-Z (Ib),
und im Falle des Vorhandenseins von zwei oder mehreren Einheiten der Formel (la) zusätzlich aus
c) 1 bis 9 divalenten Gruppen der Formel (Ic)
-CO-E-CO- (Ic),
wobei jede der gezeigten freien Valenzen in den Formeleinheiten (la) so definiert ist, daß sie unabhängig voneinander mit jeweils einer Formeleinheit (Ib) oder einer Valenz der Formeleinheit (Ic) direkt verbunden ist, und wobei in den Formeleinheiten (la) bis (Ic)
A für den Rest eines natürlichen oder modifizierten Rizinusöles oder für einen 12-Hydroxy-9-octadecenyl-(cis)- oder 12-Hydroxy-octadecyl-Rest steht;
D für eine direkte Bindung oder für einen zweiwertigen Rest eines geradkettigen oder verzweigten 2- bis 6-wertigen Alkohols mit 2 bis 8 C-Atomen oder für einen zweiwertigen Rest eines Alkylolamins HO-(CH₂)ᵥ-[NH-(CH₂)ᵤ]ₐ-NH₂, 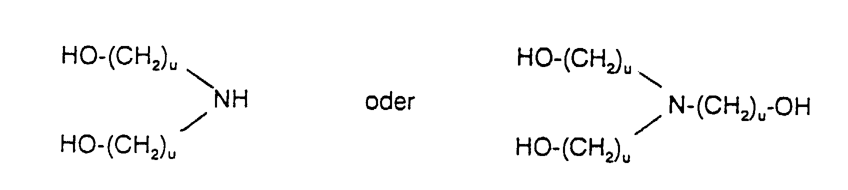 steht,
worin a für eine ganze Zahl von 0 bis 4,
u und v gleich oder verschieden sind und für eine ganze Zahl von 2 bis 14 stehen
und wobei jede oder ein Teil der OH-, NH- oder NH₂-Gruppen oxalkyliert ist;
E für einen geradkettigen, verzweigten oder cyclischen aliphatischen Rest mit 0 bis 20 Kohlenstoffatomen;
für einen aromatischen Rest mit 6 bis 10 Kohlenstoffatomen;
für einen Alkenylrest auf Basis einer dimerisierten ungesättigten C₂₈-C₇₂-Fettsäure;
oder für eine Gruppe der Formeln -CH=CH-, -CH₂CH(SO₃M)- oder -CH(SO₃M)CH₂- steht, worin M ein Kation ist;
X für eine Gruppe der Formeln -CH₂CH₂-, -CH(CH₃)CH₂ und -CH₂CH(CH₃)- oder für eine Kombination davon steht;
Z für gleiche oder verschiedene Reste Z¹ bis Z⁵ steht, worin
Z¹ Wasserstoff, oder
Z² einen Acylrest der Formel R¹-CO-, in der R¹ einen Phenyl-oder Naphthyl- rest bedeutet,
Z³ einen Acylrest einer unmodifizierten oder modifizierten natürlichen Harzsäure,
Z⁴ einen Acylrest einer Di- oder Tricarbonsäure auf Basis einer di- oder trimerisierten C₂₈-C₇₂-Fettsäure oder einen Acylrest einer gesättigten oder ungesättigten C₈-C₂₂-Fettsäure,
Z⁵ gleiche oder verschiedene Reste der Formeln -CO-(CH₂)_{q}-COOM, -CO-CH=CH-COOM, -CO-CH₂-CH(SO₃M)-COOM, -CO-CH(SO₃M)-CH₂-COOM und -OC-C₆H₄-COOM bedeuten, worin q für eine ganze Zahl von 0 bis 10 steht,
Z⁶ -SO₃M,
worin M für Wasserstoff; ein Alkalimetall; ein Äquivalent eines Erdalkalimetalls; ein Oxalkylrest der Formel (X-O-)ₙH; eine Ammoniumgruppe, die unsubstituiert oder durch ein bis vier C₁-C₅-Alkylreste oder ein bis vier C₂-C₅-Alkylolreste substituiert ist; eine aus Ammoniak oder aus C₁-C₅-Alkylaminen oder C₂-C₅-Alkylolaminen durch Anlagerung von 1 bis 150 Ethylenoxid- oder Propylenoxideinheiten oder einer Kombination von Ethylenoxid- und Propylenoxideinheiten erhaltene Ammoniumgruppe; oder eine Gruppe der Formel (II) 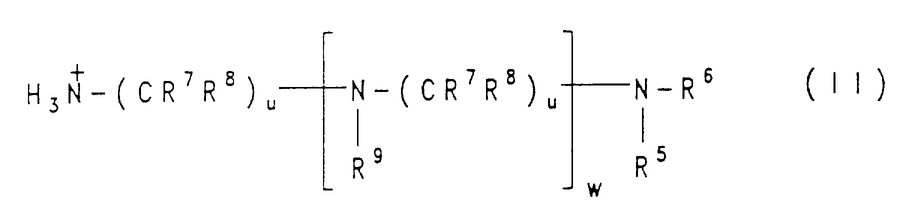 ist, worin R⁹, R⁵ und R⁶ gleich oder verschieden sind und für ein Wasserstoffatom oder eine Hydroxyalkylgruppe mit 1 bis 6 C-Atomen und R⁷ und R⁸ gleich oder verschieden sind und für Wasserstoff oder Methyl stehen, u jeweils gleich oder verschieden ist und eine ganze Zahl von 2 bis 14 bedeutet und w für eine ganze Zahl von Null bis 25 steht; oder worin M für eine Gruppe der Formel (III) steht 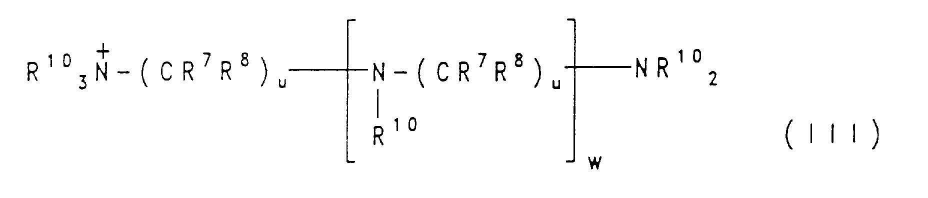 worin R¹⁰ die Gruppe H(-O-X-)_{y} bedeutet, worin X die vorstehend genannten Bedeutungen hat und y eine ganze Zahl von 1 bis 100 ist,
m im Falle, daß A ein Rizinolsäurerest ist, für die Zahl 1, und im Falle, daß A ein Rizinusölrest ist, für eine Zahl von 1 bis 2,
n für eine Zahl von 1 bis 250,
p im Falle, daß A ein Rizinusöl ist, für die Zahl 0, und im Falle, daß A ein Rizinolsäurerest ist, für die Zahl 1 und
r für eine Zahl von 1 bis 6 stehen,
und wobei mindestens ein Rest Z aus der Gruppe Z³ ist; oder wobei mindestens zwei Einheiten der Formel (la) über eine divalente Gruppe der Formel (Ic) miteinander verknüpft sind und Z die Bedeutungen von Z¹ bis Z⁶ hat.

2. Verbindung nach Anspruch 1, enthaltend 1 bis 5 divalente bis nonavalente Einheiten der Formel (la), worin
A für den Rest eines natürlichen oder modifizierten Rizinusöles steht und p daher die Zahl 0 bedeutet,
X für -CH₂CH₂- und
n für eine ganze Zahl von 5 bis 125, vorzugsweise 10 bis 80, stehen.

3. Verbindung nach Anspruch 1, enthaltend 1 bis 5 divalente bis nonavalente Einheiten der Formel (Ia), worin
A für einen 12-Hydroxy-9-octadecenyl-(cis)- oder 12-Hydroxy-octadecyl-Rest steht, p die Zahl 1,
D für einen zweiwertigen Rest eines geradkettigen oder verzweigten 2- bis 6-wertigen Alkohols mit 2 bis 6 C-Atomen oder eines C₂-C₃-Alkylolamins, C₂-C₃-Alkylol-C₂-C₃-alkylendiamins, C₂-C₃-Alkylol-di-C₂-C₃-alkylen-triamins oder C₂-C₃-Alkylol-tri-C₂-C₃-alkylen-tetramins,
X für -CH₂CH₂- und
n für eine ganze Zahl von 5 bis 125, vorzugsweise 10 bis 80, stehen.

4. Verbindung nach mindestens einem der Ansprüche 1 bis 3, enthaltend 1 bis 23, vorzugsweise 2 bis 9, monovalente Reste der Formel (Ib), worin Z gleiche oder verschiedene Reste der Formeln Z², Z³, Z⁴ und Z⁵, vorzugsweise Z³ und Z⁵, bedeutet.

5. Verbindung nach mindestens einem der Ansprüche 1 bis 4, enthaltend 1 bis 4 divalente Gruppen der Formel (Ic), worin
E für einen geradkettigen, verzweigten oder cyclischen aliphatischen Alkylenrest mit 1 bis 10 Kohlenstoffatomen, vorzugsweise Methylen, Ethylen, Propylen, Butylen, Pentylen, Hexylen und Decylen; für einen Phenylenrest; oder für eine Gruppe der Formeln -CH=CH-, -CH₂CH(SO₃M)- oder -CH(SO₃M)CH₂-, worin M ein Kation ist, steht.

6. Verbindung nach mindestens einem der Ansprüche 1 bis 5, dadurch gekennzeichnet, daß die dem Acylrest Z² zugrundeliegende Carbonsäure Benzoesäure, 1-Naphthoesäure oder 2-Naphthoesäure ist.

7. Verbindung nach mindestens einem der Ansprüche 1 bis 4, dadurch gekennzeichnet, daß die dem Acylrest Z³ zugrundeliegenden Harzsäuren diejenigen sind, die in handelsüblichen Kolophoniumarten vorliegen.

8. Verbindung nach mindestens einem der Ansprüche 1 bis 4, dadurch gekennzeichnet, daß die dem Acylrest Z⁵ zugrundeliegende Säure Oxalsäure, Malonsäure, Bernsteinsäure, Glutarsäure, Adipinsäure, Pimelinsäure, Korksäure, Acelainsäure, Sebacinsäure, Phthalsäure, Isophthalsäure, Terephthalsäure, Maleinsäure, Fumarsäure und Sulfobernsteinsäure, vorzugsweise Bernsteinsäure, Phthalsäure, Terephthalsäure, Maleinsäure und Sulfobernsteinsäure, insbesondere Sulfobernsteinsäure, ist.

9. Verbindung nach mindestens einem der Ansprüche 1 bis 5 und 8, dadurch gekennzeichnet, daß
M für Wasserstoff; ein Alkalimetall; ein Äquivalent eines Erdalkalimetalls; eine Ammoniumgruppe, die durch ein bis vier C₁-C₅-Alkylreste oder ein bis vier C₂-C₅-Alkylolreste substituiert ist; eine aus Ammoniak oder aus C₁-C₅-Alkylaminen oder C₂-C₅-Alkylolaminen durch Anlagerung von 5 bis 30 Ethylenoxid- oder Propylenoxideinheiten oder einer Kombination davon erhaltene Ammoniumgruppe; eine Gruppe der Formel (II) 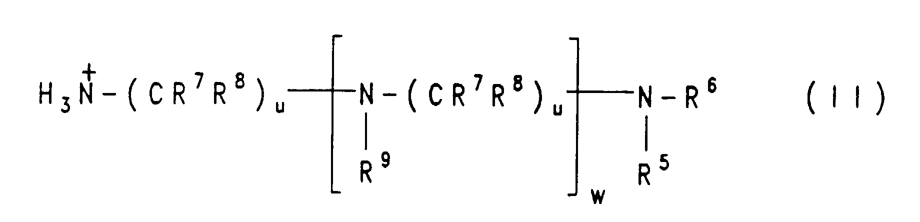 worin R⁹, R⁵ und R⁶ gleich oder verschieden sind und für ein Wasserstoffatom oder Hydroxyalkyl mit 2 bis 3 C-Atomen und R⁷ und R⁸ gleich oder verschieden sind und für Wasserstoff oder Methyl stehen, u jeweils gleich oder verschieden ist und eine ganze Zahl von 2 bis 3 bedeutet und w für eine ganze Zahl von Null bis 5 steht; oder worin M für eine Gruppe der Formel (III) steht 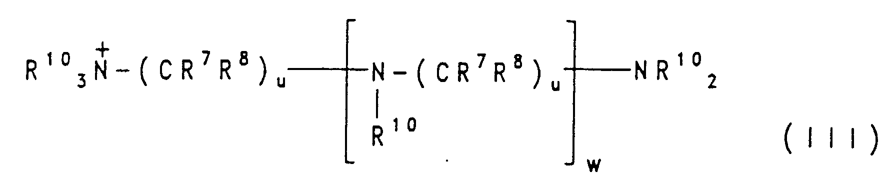 worin R¹⁰ die Gruppe H(-O-X-)_{y} bedeutet,
worin X die vorstehend genannten Bedeutungen hat und y eine ganze Zahl von 1 bis 30 ist.

10. Verfahren zur Herstellung einer Verbindung nach mindestens einem der Ansprüche 1 bis 9, dadurch gekennzeichnet, daß man
a) natürliches oder modifiziertes Rizinusöl mit mindestens einer den Resten Z², Z³, Z⁴, Z⁵, Z⁶ und -OC-E-CO- zugrundeliegenden Säure oder einem reaktiven Derivat dieser Säure teilweise oder vollständig verestert, wobei der erhaltene Ester noch mindestens eine freie Hydroxygruppe enthält, oder daß man natürliche oder hydrierte Ricinolsäure mit einem dem Rest D zugrundeliegenden Alkohol oder Alkylolamin verestert;
b) anschließend die freien Hydroxygruppen oder Carboxygruppen der nach a) erhältlichen Verbindungen mit Ethylenoxid oder Propylenoxid oder beiden Epoxiden hintereinander oder einem Gemisch beider Epoxide oxalkyliert;
c) anschließend gegebenenfalls die nach b) erhaltenen Oxalkylate mit
c1) mindestens einer den Acylresten Z², Z³, Z⁴, Z⁵ und -OC-E-CO- zugrundeliegenden Carbonsäure oder einem reaktiven Derivat dieser Carbonsäure, vorzugsweise einem Anhydrid, vollständig verestert oder teilverestert oder mit mehreren der vorstehend genannten Carbonsäuren oder deren reaktiven Derivaten mischverestert,
c2) und vorhandene Maleinsäurehalbestergruppen gegebenenfalls mit einem Sulfit oder mit schwefliger Säure umsetzt
c3) oder die nach b) erhaltenen Oxalkylate mit einem dem Rest Z⁶ zugrundeliegenden Sulfatierungsmittel, vorzugsweise Schwefelsäure, Chlorsulfonsäure, Amidosulfonsäure oder Schwefeltrioxid, sulfatiert; und
d) im Falle, daß in c) ein Rest der Formel Z⁵ oder Z⁶ eingeführt wurde, gegebenenfalls mit einer dem Rest M zugrundeliegenden Base in das entsprechende Salz oder Oxalkylat überführt.

11. Verfahren nach Anspruch 10, dadurch gekennzeichnet, daß in den Reaktionsschritten
a1) 1 bis 9 Mol, vorzugsweise 1 bis 4 Mol, einer den genannten Acylresten zugrundeliegenden Säure oder Anhydrid pro Mol Rizinusölfettkörper;
b) 1 bis 250 Mol, vorzugsweise 5 bis 125 Mol, insbesondere 10 bis 80 Mol, Ethylenoxid, Propylenoxid oder einer Kombination davon pro Mol der nach a1) erhältlichen Verbindung umgesetzt werden;
gegebenenfalls
c1) 1 bis 9 Mol, vorzugsweise 1 bis 4 Mol, einer den genannten Acylresten zugrundeliegenden Säure oder eines reaktiven Derivats dieser Säure pro Mol des nach b) erhältlichen Oxalkylats,
c2) 1 bis 1,5 Mol, vorzugsweise 1 bis 1,1 Mol, eines Sulfits oder schwefliger Säure pro Mol Maleinsäurehalbestergruppe oder
c3) 1 bis 9 Mol, vorzugsweise 1 bis 4 Mol, eines Sulfatierungsmittels pro Mol des nach b) erhältlichen Oxalkylats umgesetzt werden,
und
d) im Falle, daß in c) ein Rest der Formel Z⁵ oder Z⁶ eingeführt wurde, gegebenenfalls 1 bis 9 Mol, vorzugsweise 1 bis 4 Mol, einer dem Rest M zugrundeliegenden Base pro Mol der nach c) erhältlichen Verbindung umgesetzt werden.

12. Verfahren nach mindestens einem der Ansprüche 10 bis 11, dadurch gekennzeichnet, daß im Anschluß an den Reaktionsschritt c) ein oder mehrere Oxalkylierungsschritte analog dem Reaktionsschritt b) und gegebenenfalls ein oder mehrere Veresterungsschritte analog dem Reaktionsschritt c) durchgeführt werden.

13. Verfahren nach Anspruch 10, dadurch gekennzeichnet, daß man in den Reaktionsschritten al) oder cl) oder in den Reaktionsschritten a1) und c1) 2 bis 10, vorzugsweise 2 bis 5, Einheiten der Formel (la) durch Veresterung mit einer Dicarbonsäure der Formel HOOC-E-COOH oder mit einem dieser Dicarbonsäure zugrundeliegenden reaktiven Derivat im Molverhältnis Verbindung (Ia) : Dicarbonsäure(derivat) von 2:1 bis 10:9, vorzugsweise 2:1 bis 5:4, miteinander verknüpft.

14. Verwendung einer Verbindung nach mindestens einem der Ansprüche 1 bis 9 als oberflächenaktives Mittel.

15. Verwendung nach Anspruch 14 als Kupplungshilfs- und Präparationsmittel bei der Herstellung von Azofarbmitteln, vorzugsweise von Azopigmenten.

16. Verwendung nach Anspruch 14 als Dispergiermittel für die Herstellung von Feststoffdispersionen, vorzugsweise von anorganischen und organischen Pigmentdispersionen, zum Pigmentieren von wäßrigen Systemen, insbesondere Dispersionsanstrichfarben, wäßrige und wäßrig/alkoholische Flexodruckfarben und zum Pigmentieren von Viskose, Papiermassen und Leder sowie für die Herstellung von Gerbmitteldispersionen auf Metallsalzbasis.

17. Verwendung nach Anspruch 14 als Emulgiermittel bei der Herstellung von Flüssigemulsionen, bevorzugt Mineralöl- und Fettkörperemulsionen, Metallbearbeitungs- und Korrosionsschutzmitteln, Reinigungsverstärkern und Carrieremulsionen sowie für die Formulierung von Pflanzenschutz- und Schädlingsbekämpfungsmitteln.

18. Verwendung nach Anspruch 14 als Färbereihilfs-, Netz-, Egalisier-, Flotations-, Viskosezusatz- oder Viskoseveredlungsmittel sowie als Broschurhilfsmittel für Textilien und Leder.

## Claims

1. A compound based on a castor oil or a ricinoleic acid, consisting of
a) 1 to 10 units of the formula (Ia) 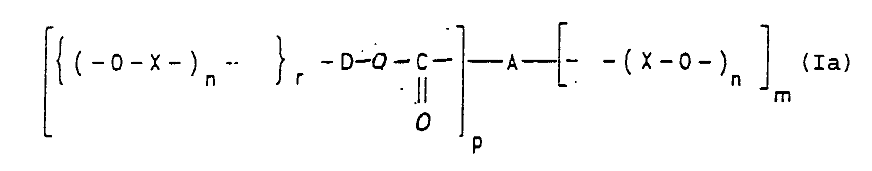
b) 1 to 72 monovalent radicals of the formula (Ib)
-Z (Ib),
and, in the case where two or more units of the formula (Ia) are present, additionally of
c) 1 to 9 divalent groups of the formula (Ic)
-CO-E-CO- (Ic),
each of the free valences shown in formula units (Ia) being defined such that each, independently of the others, is directly linked to a formula unit (Ib) or a valence of the formula unit (Ic), and, in formula units (Ia) to (Ic),
A being the radical of a natural or modified castor oil or a cis-12-hydroxy-9-octadecenyl or 12-hydroxyoctadecyl radical;
D being a direct bond or a divalent radical of a straight-chain or branched di- or hexahydric alcohol having 2 to 8 carbon atoms or a divalent radical of an alkylolamine HO-(CH₂)ᵥ-[NH-(CH₂)ᵤ]ₐ-NH₂, 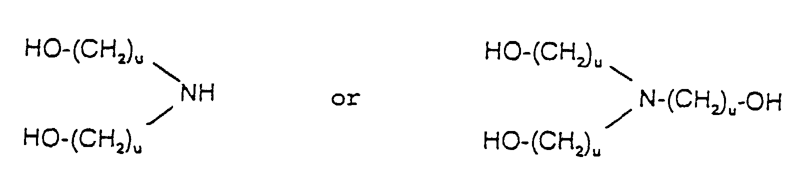 in which a is an integer from 0 to 4,
u and v are identical or different and are an integer from 2 to 14
and each or a portion of the OH, NH or NH₂ groups being alkoxylated;
E being a straight-chain, branched or cyclic aliphatic radical having 0 to 20 carbon atoms;
an aromatic radical having 6 to 10 carbon atoms; an alkenyl radical based on a dimerized unsaturated C₂₈-C₇₂-fatty acid;
or a group of the formulae -CH=CH-, -CH₂CH(SO₃M)- or -CH(SO₃M)CH₂-, in which M is a cation;
X being a group of the formulae -CH₂CH₂-, -CH(CH₃)CH₂ and -CH₂CH(CH₃)- or a combination thereof;
Z being identical or different radicals Z¹ to Z⁶, in which
Z¹ is hydrogen,
Z² is an acyl radical of the formula R¹-CO-, in which R¹ is a phenyl or naphthyl radical,
Z³ is an acyl radical of an unmodified or modified natural resin acid,
Z⁴ is an acyl radical of a di- or tricarboxylic acid based on a di- or trimerized C₂₈-C₇₂-fatty acid or an acyl radical of a saturated or unsaturated C₈-C₂₂-fatty acid,
radicals Z⁵ are identical or different radicals of the formulae -CO-(CH₂)_{q}-COOM, -CO-CH=CH-COOM, -CO-CH₂-CH(SO₃M)-COOM,-CO-CH(SO₃M)-CH₂-COOM and -OC-C₆H₄-COOM, in which q is an integer from 0 to 10,
Z⁶ is -SO₃M,
in which M is hydrogen; an alkali metal; one equivalent of an alkaline earth metal; an oxyalkyl radical of the formula (X-O-)ₙH; an ammonium group which is unsubstituted or substituted by one to four C₁-C₅-alkyl radicals or one to four C₂-C₅-alkylol radicals; an ammonium group obtained from ammonia or from C₁-C₅-alkylamines or C₂-C₅-alkylolamines by an addition reaction with 1 to 150 ethylene oxide or propylene oxide units or a combination of ethylene oxide and propylene oxide units; or a group of the formula (II) 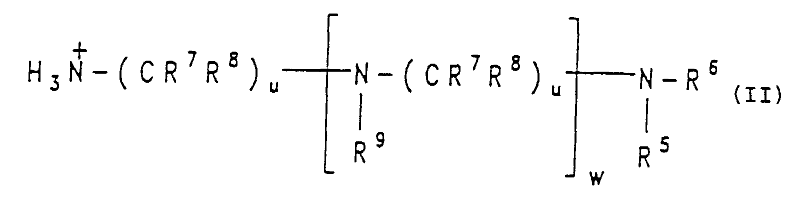 in which R⁹, R⁵ and R⁶ are identical or different and are a hydrogen atom or a hydroxyalkyl group having 1 to 6 carbon atoms and R⁷ and R⁸ are identical or different and are hydrogen or methyl, each u is identical to or different from the others and is an integer from 2 to 14 and w is an integer from zero to 25; or in which M is a group of the formula (III) 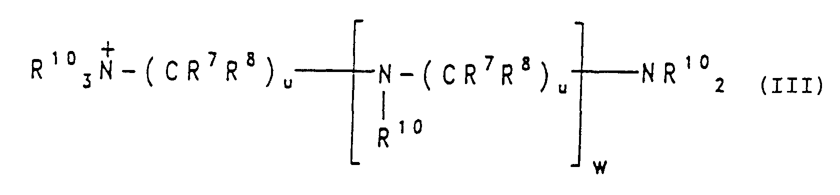 in which R¹⁰ is the group H-(-O-X-)_{y}, in which X has the abovementioned meanings and y is an integer from 1 to 100,
m, in the case where A is a ricinoleic acid radical, being 1, and, in the case where A is a castor oil radical, being a number from 1 to 2,
n being a number from 1 to 250,
p, in the case where A is a castor oil, being 0, and, in the case where A is a ricinoleic acid radical, being 1, and
r being a number from 1 to 6,
and at least one radical Z being from the group Z³; or at least two units of the formula (Ia) being linked to one another via a divalent group of the formula (Ic) and Z having the meanings of Z¹ to Z⁶.

2. A compound as claimed in claim 1, which contains 1 to 5 divalent to nonavalent units of the formula (Ia), in which
A is the radical of a natural or modified castor oil and p is therefore the number 0,
X is -CH₂CH₂- and
n is an integer from 5 to 125, preferably 10 to 80.

3. A compound as claimed in claim 1, which contains 1 to 5 divalent to nonavalent units of the formula (Ia), in which
A is a cis-12-hydroxy-9-octadecenyl or 12-hydroxyoctadecyl radical, p is the number 1,
D is a divalent radical of a straight-chain or branched di- to hexahydric alcohol having 2 to 6 carbon atoms or of a C₂-C₃-alkylolamine, C₂-C₃-alkylol-C₂-C₃-alkylenediamine, C₂-C₃-alkylol-di-C₂-C₃-alkylenetriamine or C₂-C₃-alkyloltri-C₂-C₃-alkylenetetramine,
X is -CH₂CH₂- and
n is an integer from 5 to 125, preferably 10 to 80.

4. A compound as claimed in at least one of claims 1 to 3, which contains 1 to 23, preferably 2 to 9, monovalent radicals of the formula (Ib), in which Z is identical or different radicals of the formulae Z², Z³, Z⁴ and Z⁵, preferably Z³ and Z⁵.

5. A compound as claimed in at least one of claims 1 to 4, which contains 1 to 4 divalent groups of the formula (Ic), in which
E is a straight-chain, branched or cyclic aliphatic alkylene radical having 1 to 10 carbon atoms, preferably methylene, ethylene, propylene, butylene, pentylene, hexylene and decylene; a phenylene radical; or a group of the formulae -CH=CH-, -CH₂CH(SO₃M)- or -CH(SO₃M)CH₂-, in which M is a cation.

6. A compound as claimed in at least one of claims 1 to 5, wherein the carboxylic acid on which the acyl radical Z² is based is benzoic acid, 1-naphthoic acid or 2-naphthoic acid.

7. A compound as claimed in at least one of claims 1 to 4, wherein the resin acids on which the acyl radical Z³ is based are those present in commercially available rosin types.

8. A compound as claimed in at least one of claims 1 to 4, wherein the acid on which the acyl radical Z⁵ is based is oxalic acid, malonic acid, succinic acid, glutaric acid, adipic acid, pimelic acid, suberic acid, azelaic acid, sebacic acid, phthalic acid, isophthalic acid, terephthalic acid, maleic acid, fumaric acid and sulfosuccinic acid, preferably succinic acid, phthalic acid, terephthalic acid, maleic acid and sulfosuccinic acid, in particular sulfosuccinic acid.

9. A compound as claimed in that least one of claims 1 to 5 and 8, wherein
M is hydrogen; an alkali metal; one equivalent of an alkaline earth metal; an ammonium group which is substituted by one to four C₁-C₅-alkyl radicals or one to four C₂-C₅-alkylol radicals; an ammonium group obtained from ammonia or from C₁-C₅-alkylamines or C₂-C₅-alkylolamines by an addition reaction with 5 to 30 ethylene oxide or propylene oxide units or a combination thereof; a group of the formula (II) 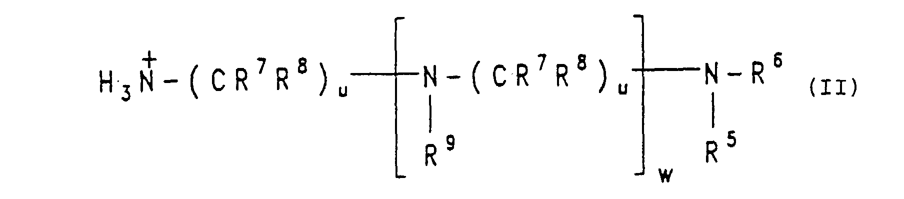
in which R⁹, R⁵ and R⁶ are identical or different and are a hydrogen atom or hydroxyalkyl having 2 to 3 carbon atoms and R⁷ and R⁸ are identical or different and are hydrogen or methyl, each u is identical to or different from the others and is an integer from 2 to 3 and w is an integer from zero to 5; or in which M is a group of the formula (III) 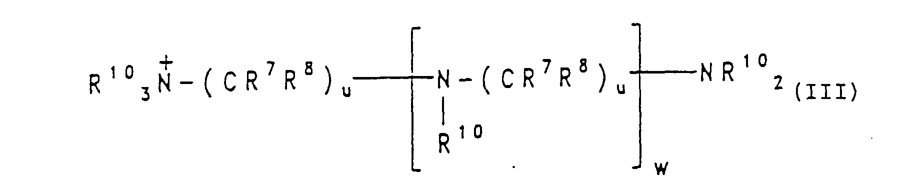
in which R¹⁰ is the group H(-O-X-)_{y},
in which X has the abovementioned meanings and y is an integer from 1 to 30.

10. A process for the preparation of a compound as claimed in at least one of claims 1 to 9, which comprises
a) esterifying natural or modified castor oil partially or completely with at least one acid on which the radicals Z², Z³, Z⁴, Z⁵, Z⁶ and -OC-E-CO- are based or a reactive derivative of this acid, the ester obtained still containing at least one free hydroxyl group, or esterifying natural or hydrogenated ricinoleic acid with an alcohol or alkylolamine on which the radical D is based;
b) then alkoxylating the free hydroxyl groups or carboxyl groups of the compounds obtainable by a) with ethylene oxide or propylene oxide or both epoxides in succession or a mixture of both epoxides;
c) then, if desired, esterifying the alkoxylates obtained by b) partially or completely with
c1) at least one carboxylic acid on which the acyl radicals Z², Z³, Z⁴, Z⁵ and -OC-E-CO- are based or a reactive derivative of this carboxylic acid, preferably an anhydride, or subjecting them to mixed esterification with a plurality of the abovementioned carboxylic acids or reactive derivatives thereof,
c2) and reacting any maleic monoester groups present, if desired, with a sulfite or with sulfurous acid
c3) or sulfating the alkoxylates obtained by b) with a sulfating agent on which the radical Z⁶ is based, preferably sulfuric acid, chlorosulfonic acid, sulfamic acid or sulfur trioxide; and
d), in the case where in c) a radical of the formula Z⁵ or Z⁶ has been introduced, converting, if desired, the product into the corresponding salt or alkoxylate with a base on which the radical M is based.

11. The process as claimed in claim 10, wherein in reaction steps
a ) 1 to 9 mol, preferably 1 to 4 mol, of an acid or anhydride on which the acyl radicals mentioned are based are reacted per mole of castor oil fatty substance;
b) 1 to 250 mol, preferably 5 to 125 mol, in particular 10 to 80 mol, of ethylene oxide, propylene oxide or a combination thereof are reacted per mole of the compound obtainable by a ); if desired
c1) 1 to 9 mol, preferably 1 to 4 mol, of an acid on which the acyl radicals mentioned are based or of a reactive derivative of this acid are reacted per mole of the alkoxylate obtainable by b),
c2) 1 to 1.5 mol, preferably 1 to 1.1 mol, of a sulfite or of sulfurous acid are reacted per mole of maleic monoester group or
c3) 1 to 9 mol, preferably 1 to 4 mol, of a sulfating agent are reacted per mole of the alkoxylate obtainable by b),
and
d), in the case where in c) a radical of the formula Z⁵ or Z⁶ has been introduced, 1 to 9 mol, preferably 1 to 4 mol, of a base on which the radical M is based are, if desired, reacted per mole of the compound obtainable by c).

12. The process as claimed in at least one of claims 10 to 11, wherein following reaction step c) one or more alkoxylation steps analogous to reaction step b) and, if desired, one or more esterification steps analogous to reaction step c) are carried out.

13. The process as claimed in claim 10, wherein in reaction steps a ) or c1) or in reaction steps a ) and c1) 2 to 10, preferably 2 to 5, units of the formula (Ia) are linked to one another by esterification with a dicarboxylic acid of the formula HOOC-E-COOH or with a reactive derivative on which this dicarboxylic acid is based in a compound (Ia) : dicarboxylic acid (derivative) molar ratio of 2:1 to 10:9, preferably 2:1 to 5:4.

14. Use of a compound as claimed in at least one of claims 1 to 9 as surface-active agent.

15. Use as claimed in claim 14 as coupling auxiliary and preparing agent in the preparation of azo colorants, preferably of azo pigments.

16. Use as claimed in claim 14 as dispersing agent for the preparation of solid dispersions, preferably of inorganic and organic pigment dispersions, for the pigmenting of aqueous systems, in particular emulsion paints, aqueous and aqueous-alcoholic flexographic printing inks and for the pigmenting of viscose, paper materials and leather and for the preparation of tanning agent dispersions based on metal salts.

17. Use as claimed in claim 14 as emulsifying agent in the preparation of liquid emulsions, preferably mineral oil emulsions and fatty substance emulsions, metal-working agents and anticorrosives, cleaning promoters and carrier emulsions and for the formulation of crop protection and pest control agents.

18. Use as claimed in claim 14 as dyeing auxiliaries, wetting agents, leveling agents, flotation agents, viscose additives or viscose finishing agents and as wetting-back auxiliaries for textiles and leathers.

## Revendications

1. Composé à base d'une huile de ricin ou d'un acide ricinoléique, constitué de
a) 1 à 10 motifs de formule (la) 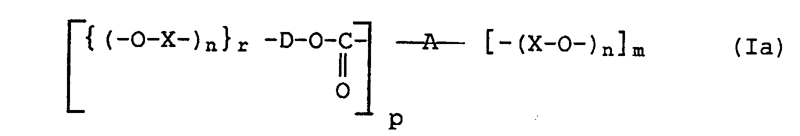
b) 1 à 72 groupes monovalents de formule (Ib)
-Z (Ib),
et dans le cas de la présence de deux ou plusieurs motifs de formule (la) en plus de
c) 1 à 9 groupes divalents de formule (Ic)
-CO-E-CO- (Ic),
dans lequel chacune des valences libres qui se présente dans les motifs de formule (la) est définie de manière à ce qu'elle soit, indépendamment des autres, liée directement avec respectivement un motif de formule (Ib) ou une valence du motif de formule (Ic), et dans lequel dans les motifs de formules (la) à (Ic)
A représente le résidu d'une huile de ricin naturelle ou modifiée ou un groupe 12-hydroxy-9-octadécényl-(cis)- ou 12-hydroxy-octadécyle;
D représente une simple liaison ou un radical divalent d'un alcool di à hexafonctionnel à chaîne linéaire ou ramifiée avec 2 à 8 atomes de carbone ou un résidu divalent d'une alkylolamine HO-(CH₂)ᵥ-[NH-(CH₂)ᵤ]ₐ-NH₂, 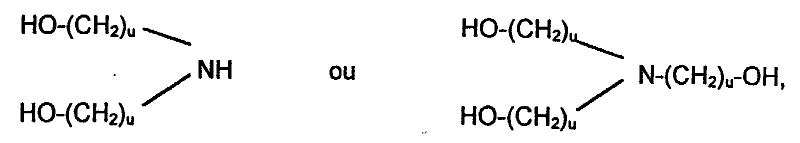 formules dans lesquelles a représente un nombre entier de 0 à 4,
u et v sont identiques ou différents et représentent un nombre entier de 2 à 14,
et dans lesquelles chacun ou une partie des groupes OH, NH ou NH₂ est alcoxylé;
E représente un groupe à chaîne linéaire, ramifiée ou cyclique aliphatique avec 0 à 20 atomes de carbone; un groupe aromatique avec 6 à 10 atomes de carbone; un groupe alcényle à base d'un acide gras en C₂₈-C₇₂ insaturé dimérisé;
ou un groupe de formules -CH=CH-, -CH₂CH(SO₃M)- ou -CH(SO₃M)CH₂-, dans lesquelles M est un cation;
X représente un groupe de formules -CH₂CH₂-, -CH(CH₃)CH₂ et -CH₂CH(CH₃)- ou une combinaison de ceux-ci;
Z représente des groupes Z¹ à Z⁶ identiques ou différents, dans lesquels
Z¹ représente un atome d'hydrogène,
Z² représente un groupe acyle de formule R¹-CO-, dans laquelle R¹ représente un groupe phényle ou naphtyle,
Z³ représente un groupe acyle d'un acide résinique naturel modifié ou non modifié,
Z⁴ représente un groupe acyle d'un acide di ou tricarboxylique à base d'un acide gras di ou trimérisé en C₂₈-C₇₂ ou un groupe acyle d'un acide gras en C₈-C₂₂ saturé ou insaturé,
Z⁵ représente des groupes identiques ou différents de formules
-CO-(CH₂)_{q}-COOM, -CO-CH=CH-COOM,
-CO-CH₂-CH(SO₃M)-COOM, -CO=CH(SO₃M)-CH₂-COOM et
-OC-C₆H₄-COOM, dans lesquelles q représente un nombre entier de O à 10,
Z⁶ représente SO₃M,
formules dans lesquelles M représente un atome d'hydrogène, un métal alcalin, un équivalent d'un métal alcalino-terreux, un groupe alcoxy de formule (X-O)ₙH, un groupe ammonium qui est non substitué ou substitué par un à quatre radicaux alkyle en C₁-C₅ ou par un à quatre radicaux alkylol en C₂-C₅, un groupe ammonium obtenu à partir de l'ammoniac ou d'alkylamines en C₁-C₅ ou d'alkylolamines en C₂-C₅ par fixation par addition de 1 à 150 motifs d'oxyde d'éthylène ou d'oxyde de propylène ou d'une combinaison de motifs d'oxyde d'éthylène et d'oxyde de propylène; ou est un groupe de formule (II) 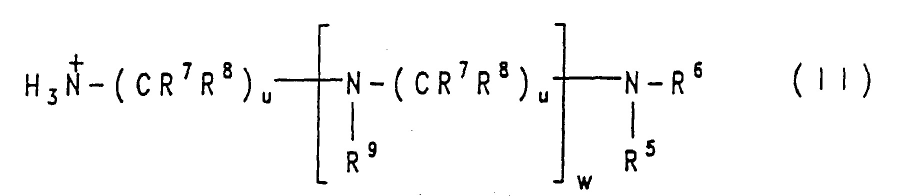 dans laquelle R⁹, R⁵ et R⁶ sont identiques ou différents et représentent un atome d'hydrogène ou un groupe hydroxyalkyle avec 1 à 6 atomes de carbone, et R⁷ et R⁸ sont identiques ou différents et représentent un atome d'hydrogène ou un groupe méthyle, u est respectivement identique ou différent et représente un nombre entier de 2 à 14, et w représente un nombre entier de zéro à 25, ou dans laquelle M représente un groupe de formule (III) 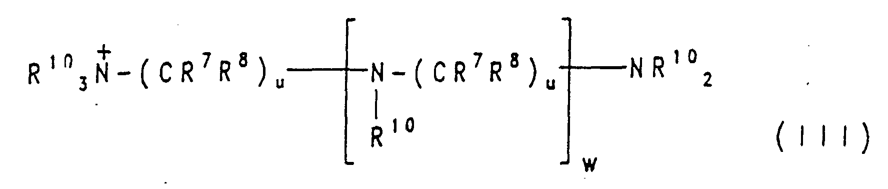 dans laquelle R¹⁰ représente le groupe H-(O-X-)_{y}, dans laquelle X a les significations citées précédemment et y est un nombre entier de 1 à 100,
m dans le cas où A est un résidu d'acide ricinoléique représente le nombre 1, et dans le cas où A est un résidu d'huile de ricin, représente un nombre de 1 à 2,
n représente un nombre de 1 à 250,
p dans le cas où A est une huile de ricin, représente le nombre 0, et dans le cas où A est un résidu d'acide ricinoléique, représente le nombre 1 et
r représente un nombre de 1 à 6,
et dans lequel au moins un groupe Z est issu du groupe Z³; ou dans lequel au moins deux motifs de formule (la) sont couplés l'un à l'autre par un groupe divalent de formule (Ic) et Z a les significations de Z¹ à Z⁶.

2. Composé selon la revendication 1, contenant 1 à 5 motifs divalents à nonavalents de formule (la), dans laquelle
A représente le résidu d'une huile de ricin naturelle ou modifiée et p signifie alors le nombre 0,
X représente -CH₂CH₂- et
n représente un nombre entier de 5 à 125, de préférence de 10 à 80.

3. Composé selon la revendication 1, contenant 1 à 5 motifs divalents à nonavalents de formule (Ia), dans laquelle
A représente un groupe 12-hydroxy-9-octadécényl-(cis)- ou 12-hydroxy-octadécyle, p représente le nombre 1,
D représente un résidu divalent d'un alcool di à hexa-fonctionnel à chaîne linéaire ou ramifiée avec 2 à 6 atomes de carbone ou d'une alkylolamine en C₂-C₃, d'une (alkylol en C₂-C₃)-(alkylène en C₂-C₃)-diamine, d'une (alkylol en C₂-C₃)-(dialkylène en C₂-C₃)-triamine ou (alkylol en C₂-C₃)-(trialkylène en C₂-C₃)-tétramine,
X représente -CH₂CH₂- et
n représente un nombre entier de 5 à 125, de préférence de 10 à 80.

4. Composé selon au moins l'une des revendications 1 à 3, contenant 1 à 23, de préférence 2 à 9, groupes monovalents de formule (Ib), dans laquelle Z représente des groupes identiques ou différents de formules Z², Z³, Z⁴ et Z⁵, de préférence Z³ et Z⁵.

5. Composé selon au moins l'une des revendications 1 à 4, contenant 1 à 4 groupes divalents de formule (Ic), dans laquelle
E représente un groupe alkylène à chaîne linéaire, ramifiée ou cyclique aliphatique avec 1 à 10 atomes de carbone; de préférence le méthylène, l'éthylène, le propylène, le butylène, le pentylène, l'hexylène et le décylène; un groupe phénylène; ou un groupe des formules -CH=CH-, -CH₂CH(SO₃M)- ou -CH(SO₃M)CH₂-, dans laquelle M est un cation.

6. Composé selon au moins l'une des revendications 1 à 5, caractérisé en ce que l'acide carboxylique correspondant au groupe acyle Z² est l'acide benzoïque, l'acide 1-naphtoïque ou l'acide 2-naphtoïque.

7. Composé selon au moins l'une des revendications 1 à 4, caractérisé en ce que les acides résiniques correspondant au groupe acyle Z³ sont ceux qui existent dans les types de colophane usuelle du commerce.

8. Composé selon au moins l'une des revendications 1 à 4, caractérisé en ce que l'acide correspondant au groupe acyle Z⁵ est l'acide oxalique, l'acide malonique, l'acide succinique, l'acide glutarique, l'acide adipique, l'acide pimélique, l'acide subérique, l'acide acélaique, l'acide sébacique l'acide phtalique, l'acide isophtalique, l'acide téréphtalique, l'acide maléique, l'acide fumarique et l'acide sulfosuccinique, de préférence l'acide sulfosuccinique, l'acide phtalique, l'acide téréphtalique, l'acide maléique, et l'acide sulfosuccinique, en particulier l'acide sulfosuccinique.

9. Composé selon au moins l'une des revendications 1 à 5 et 8, caractérisé en ce que
M représente un atome d'hydrogène; un métal alcalin; un équivalent d'un métal alcalino-terreux; un groupe ammonium qui est substitué par un à quatre radicaux alkyle en C₁-C₅ ou par un à quatre radicaux alkylol en C₂-C₅; un groupe ammonium obtenu à partir de l'ammoniac ou d'alkylamines en C₁-C₅ ou d'alkylolamines en C₂-C₅ par fixation par addition de 5 à 30 motifs d'oxyde d'éthylène ou d'oxyde de propylène ou d'une combinaison de ceux-ci; un groupe de formule (II) 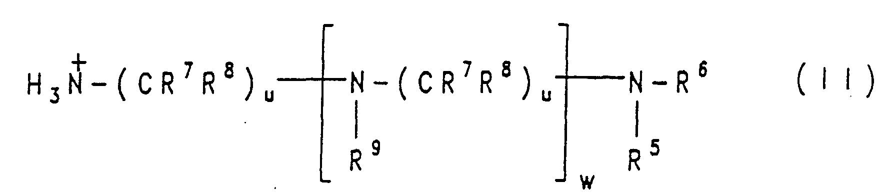 dans laquelle R⁹, R⁵ et R⁶ sont identiques ou différents et représentent un atome d'hydrogène ou un groupe hydroxyalkyle avec 2 à 3 atomes de carbone et R⁷ et R⁸ sont identiques ou différents et représentent un atome d'hydrogène ou un groupe méthyle, u est respectivement identique ou différent et représente un nombre entier de 2 à 3, et w représente un nombre entier de zéro à 5, ou dans laquelle M représente un groupe de formule (III) 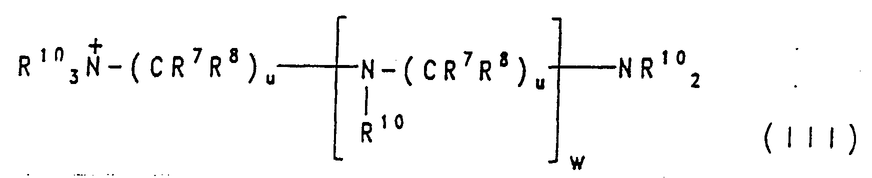 dans laquelle R¹⁰ représente le groupe H-(O-X-)y, dans laquelle X a les significations citées ci-dessus et y est un nombre entier de 1 à 30.

10. Procédé de préparation d'un composé selon au moins l'une des revendications 1 à 9, caractérisé en ce que
a) l'on estérifie totalement ou partiellement de l'huile de ricin naturelle ou modifiée avec au moins un acide correspondant aux groupes Z², Z³, Z⁴, Z⁵, Z⁶ et -OC-E-CO- ou avec un dérivé réactif de cet acide, l'ester obtenu contenant encore au moins un groupe hydroxy libre, ou en ce qu'on estérifie un acide ricinoléique naturel ou hydrogéné avec un alcool ou une alkylolamine correspondant au groupe D;
b) on réalise ensuite l'alcoxylation des groupes hydroxy ou carboxy libres des liaisons obtenues selon a) avec de l'oxyde d'éthylène ou de l'oxyde de propylène ou les deux époxydes l'un après l'autre ou avec un mélange des deux époxydes;
c) on traite éventuellement ensuite les alcoxylates obtenus selon b)
c1) par estérification totale ou partielle avec au moins un des groupes acyle Z², Z³, Z⁴, Z⁵, Z⁶ et -CO-E-CO- ou avec un dérivé réactif de cet acide carboxylique, de préférence un anhydride ou par estérification mixte avec plusieurs des acides carboxyliques cités précédemment ou de leurs dérivés réactifs,
c2) et par réaction des groupes d'hémi-ester de l'acide maléique présents éventuellement avec un sulfite ou avec de l'acide sulfureux
c3) ou par sulfatation des alcoxylates obtenus selon b) avec un agent de sulfatation correspondant au groupe Z⁶, de préférence l'acide sulfurique, l'acide chlorosulfurique, l'acide amidosulfonique ou le trioxyde de soufre; et
d) au cas où on aurait introduit en c) un groupe de formule Z⁵ ou Z⁶, en le transformant éventuellement en sel ou en alcoxylate correspondant avec une base correspondant au groupe M.

11. Procédé selon la revendication 10, caractérisé en ce qu'on met à réagir dans les étapes réactionnelles
a ) 1 à 9 moles, de préférence 1 à 4 moles, d'un acide correspondant aux groupes acyle mentionnés, ou d'un anhydride, par mole de corps gras d'huile de ricin;
b) 1 à 250 moles, de préférence 5 à 125 moles, en particulier 10 à 80 moles, d'oxyde d'éthylène, d'oxyde de propylène ou d'une combinaison de ceux-ci par mole du composé qu'on peut obtenir selon a );
éventuellement on met à réagir
c1) 1 à 9 moles, de préférence 1 à 4 moles, d'un acide correspondant aux groupes acyle cités, ou d'un dérivé réactif de cet acide, par mole de alcoxylate qu'on peut obtenir selon b);
c2) 1 à 1,5 moles, de préférence 1 à 1,1 moles, d'un sulfite ou d'acide sulfureux par mole de groupe hémi-ester d'acide maléique ou
c3) 1 à 9 moles, de préférence 1 à 4 moles, d'un agent de sulfatation par mole de l'alcoxylate qu'on peut obtenir selon b);
et on met à réagir
d) dans le cas où on a introduit en c) un groupe de formule Z⁵ ou Z⁶, éventuellement 1 à 9 moles, de préférence 1 à 4 moles, d'une base correspondant au groupe M par mole du composé qu'on peut obtenir selon c).

12. Procédé selon au moins l'une des revendications 10 à 11, caractérisé en ce que, à la suite de l'étape de réaction c), on réalise une ou plusieurs étapes d'alcoxylation analogues à l'étape de réaction b) et éventuellement une ou plusieurs étapes d'estérification analogues à l'étape de réaction c).

13. Procédé selon la revendication 10, caractérisé en ce qu'on couple entre eux dans les étapes de réaction a ) ou cI) ou dans les étapes de réaction a ) et cI) 2 à 10, de préférence 2 à 5, motifs de formule (la) par estérification avec un acide dicarboxylique de formule HOOC-E-COOH ou avec un dérivé réactif correspondant à cet acide dicarboxylique dans le rapport molaire composé (Ia):acide (dérivé) dicarboxylique de 2:1 à 10:9, de préférence de 2:1 à 5:4.

14. Utilisation d'un composé selon au moins l'une des revendications 1 à 9 comme agent tensioactif.

15. Utilisation selon la revendication 14 comme agent de copulation et de préparation pour la fabrication de colorants azoïques, de préférence de pigments azoïques.

16. Utilisation selon la revendication 14 comme agent dispersant pour la fabrication de dispersions de colorants, de préférence de dispersions de pigments inorganiques et organiques, pour la pigmentation de systèmes aqueux, en particulier de colorants de peintures dispersables, de colorants en solution aqueuse/alcoolique pour la flexographie et pour la pigmentation de viscose, de pâtes à papier et de cuir ainsi que pour la préparation de dispersions d'agents de tannage à base de sels métalliques.

17. Utilisation selon la revendication 14 comme agent émulsionnant pour la fabrication d'émulsions liquides, de préférence d'émulsions d'huile minérale et de corps gras, d'agents anticorrosion et pour l'usinage des métaux, de renforçateurs de nettoyage et d'émulsions d'agent véhiculaires ainsi que pour la formulation d'agents phytosanitaires et de lutte contre les nuisibles.

18. Utilisation selon la revendication 14 comme additif de coloration, agent mouillant, égalisant, agent de flottation, additif de la viscose, agent de transformation de la viscose ainsi que comme adjuvants de brochure pour les textiles et le cuir.
